# EUROPEAN PATENT APPLICATION

(11) **EP 3 936 613 A1**
(43) Date of publication of application: **12.01.2022**
(21) Application number: 20766317.0
(22) Date of filing: 06.03.2020
(51) Int. Cl.: C12N 15/09, C12Q 1/6827, C12Q 1/6886

(54) **METHOD FOR DETERMINING SIDE EFFECTS OF TRASTUZUMAB AND KIT FOR SAME**

(30) Priority: 07.03.2019 JP 2019041127
(71) Applicant: Toyo Kohan Co., Ltd., Tokyo 141-8260 (JP); Japanese Foundation For Cancer Research, Tokyo 135-8550 (JP)
(72) Inventor: ZEMBUTSU Hitoshi, Tokyo 135-8550 (JP); UDAGAWA Chihiro, Tokyo 135-8550 (JP); NAKANO Mari, Kawasaki-shi, Kanagawa 216-8511 (JP)
(74) Representative: Wohlfahrt, Jan Günther
(86) International application number: PCT/JP2020/009707
(87) International publication number: WO 2020/179913

(57) **Abstract**

A side effect of trastuzumab is predicted by using a gene polymorphism associated with the side effect of trastuzumab. The genotype of a gene polymorphism specified by one selected from the group consisting of rs9316695, rs11932853, rs28415722, rs7406710 and rs8032978 is determined and a side effect from administration of trastuzumab is determined based on the genotype determined.

## Description

### Technical Field

The present invention relates to a method for determining side effects of trastuzumab and a kit for the method.

### Background Art

Trastuzumab is an anti-malignant tumor agent (trade name: Herceptin) containing a humanized monoclonal antibody targeting human epidermal growth factor receptor type 2 (HER2, also known as c-erbB-2) as a main component. HER2 is found to be overexpressed in about 25 to 30% of the patients with metastatic breast cancer. It is known that the growth of cancer cells is promoted by overexpression of HER2, and that prognosis for patients with tumors having HER2 overexpression is poor. Currently, trastuzumab can be administered to patients with breast cancer which is observed to have overexpression of HER2 or patients with unresectable and progressive/recurrent stomach cancer which is observed to have overexpression of HER2.

Examples of a side effect from administration of trastuzumab include heart disorders: heart failure (signs: e.g., dyspnea, orthopnea, cough, symptom/abnormality: e.g., S3 gallop, reduction in ejection fraction, peripheral edema), cardiogenic shock, pulmonary edema, pericardial effusion, cardiomyopathy, pericarditis, arrhythmia, and bradycardia. Accordingly, in administration of trastuzumab, the conditions of patients (including a change in left ventricular ejection fraction (LVEF)) must be closely monitored by cardiac function tests (e.g., echocardiography) depending on the expression or degree of severity of cardiac symptoms. As described, trastuzumab is known to have cardiotoxicity.

Conventionally, in view of a side effect of trastuzumab cardiotoxicity mentioned above, it has been recommended that trastuzumab is carefully administered to patients on medication of anthracycline drugs or patients with history thereof; patients receiving a radiation therapy to the chest; patients with heart failure or a history thereof; patients with declined left ventricular ejection fraction (LVEF); patients with uncontrollable arrhythmias; patients with severe valvular heart diseases; patients with coronary artery disease (e.g., myocardial infarction, angina) or a history thereof; patients with hypertension or a history thereof; patients with resting dyspnea (caused by, e.g., lung metastases, cardiovascular disease) or a history thereof; or aged individuals.

As an index for predicting a side effect before administration of trastuzumab, a gene polymorphism specified by rs139944387 present in the EYS gene is known, as disclosed in Non Patent Literature 1. According to Non Patent Literature 1, whether a side effect of trastuzumab is developed or not can be predicted by detecting a mutant of gene polymorphism specified by rs139944387 as a risk allele. In Non Patent Literature 1, case-control-related analysis of about 2000 gene mutations was conducted to specify rs139944387 associated with development of a side effect of trastuzumab from among the gene mutations.

### Citation List

### Non Patent Literature

Non Patent Literature 1: Cancer Science. 2018; 109: 446-452

### Summary of Invention

### Technical Problem

In investigating characteristics (phenotypes) of individuals by using gene polymorphism, use of a plurality of gene polymorphisms is sometimes better than use of a single gene polymorphism in view of improvement of accuracy. Side effects of trastuzumab can be more accurately evaluated by using a plurality of gene polymorphisms.

Accordingly, an object of the present invention is to provide a method of specifying a gene polymorphism associated with a side effect of trastuzumab and predicting the side effect of trastuzumab by using the gene polymorphism.

### Solution to Problem

The present inventors conducted intensive studies with a view to attaining the above object. As a result, they found a plurality of gene polymorphisms associated with side effects of trastuzumab and accomplished the present invention. The present invention encompasses the following.

(1) A method comprising steps of: analyzing a gene polymorphism specified by one selected from the group consisting of rs9316695, rs11932853, rs28415722, rs7406710 and rs8032978, or a gene polymorphism in linkage disequilibrium or genetic linkage with the gene polymorphism present in genomic DNA of a biological sample taken from a subject; determining the genotype of the gene polymorphism; and determining a side effect from administration of trastuzumab based on the determined genotype.
(2) The method according to (1), wherein the gene polymorphism specified by rs9316695 is located on a long arm of chromosome 13 (13q14.3) and is a single nucleotide polymorphism having cytosine as wild-type and adenine as mutant.
(3) The method according to (1), wherein, in the gene polymorphism specified by rs9316695, a mutant is a risk allele; and it is determined that a possibility of developing a side effect of trastuzumab is low in a case of wild-type homozygosity, a possibility of developing a side effect of trastuzumab is high in a case of mutant/wild-type heterozygosity, and a possibility of developing a side effect of trastuzumab is higher in a case of mutant homozygosity.
(4) The method according to (1), wherein the gene polymorphism specified by rs11932853 is located on a long arm of chromosome 4 (4q25) and is a single nucleotide polymorphism having thymine as wild-type and cytosine as mutant.
(5) The method according to (1), wherein, in the gene polymorphism specified by rs11932853, a wild-type is a risk allele; and it is determined that a possibility of developing a side effect of trastuzumab is low in a case of mutant homozygosity, a possibility of developing a side effect of trastuzumab is high in a case of mutant/wild-type heterozygosity, and a possibility of developing a side effect of trastuzumab is higher in a case of mutant homozygosity.
(6) The method according to (1), wherein the gene polymorphism specified by rs28415722 is located on a long arm of chromosome 15 (15q26.3) and is a single nucleotide polymorphism having guanine as wild-type and adenine as mutant.
(7) The method according to (1), wherein, in the gene polymorphism specified by rs28415722, a mutant is a risk allele; and it is determined that a possibility of developing a side effect of trastuzumab is low in a case of wild-type homozygosity, a possibility of developing a side effect of trastuzumab is low in a case of mutant/wild-type heterozygosity, and a possibility of developing a side effect of trastuzumab is higher in a case of mutant homozygosity.
(8) The method according to (1), wherein the gene polymorphism specified by rs7406710 is located on a long arm of chromosome 17 (17q25.3) and is a single nucleotide polymorphism having cytosine as wild-type and thymine as mutant.
(9) The method according to (1), wherein, in the gene polymorphism specified by rs7406710, a wild-type is a risk allele; and it is determined that a possibility of developing a side effect of trastuzumab is low in a case of mutant homozygosity, a possibility of developing a side effect of trastuzumab is low in a case of mutant/wild-type heterozygosity, and a possibility of developing a side effect of trastuzumab is higher in a case of wild-type homozygosity.
(10) The method according to (1), wherein the gene polymorphism specified by rs8032978 is located on a long arm of chromosome 15 (15q26.3) and is a single nucleotide polymorphism having adenine as wild-type and guanine as mutant.
(11) The method according to (1), wherein, in the gene polymorphism specified by rs8032978, a mutant is a risk allele; and it is determined that a possibility of developing a side effect of trastuzumab is low in a case of wild-type homozygosity, a possibility of developing a side effect of trastuzumab is high in a case of mutant/wild-type heterozygosity, and a possibility of developing a side effect of trastuzumab is higher in a case of mutant homozygosity.
(12) The method according to (1), wherein the subject is a patient with cancer which is observed to have overexpression of HER2.
(13) The method according to (12), wherein the cancer is breast cancer or stomach cancer.
(14) The method according to (1), wherein the side effect is at least one selected from heart failure, cardiogenic shock, pulmonary edema, pericardial effusion, cardiomyopathy, pericarditis, arrhythmia and bradycardia.
(15) A probe set for determining a side effect from administration of trastuzumab, comprising an oligonucleotide that hybridizes, under stringent conditions, with a region of consecutive 5 to 50 nucleotides containing a gene polymorphism specified by one selected from the group consisting of rs9316695, rs11932853, rs28415722, rs7406710 and rs8032978, or a gene polymorphism in linkage disequilibrium or genetic linkage with the gene polymorphism.
(16) The probe set for determining a side effect according to (15), comprising a wild-type probe corresponding to a wild-type in the gene polymorphism and a mutant probe corresponding to a mutant in the gene polymorphism.

The probe set for determining a side effect from administration of trastuzumab according to the present invention may be a kit for determining a side effect of trastuzumab, comprising primers for amplifying the region of 5 to 50 nucleotides contained in a sample. Specifically, the probe set according to the present invention may comprise primers specifically amplifying the region of consecutive 5 to 50 nucleotides containing a gene polymorphism specified by one selected from the group consisting of rs9316695, rs11932853, rs28415722, rs7406710 and rs8032978, or a gene polymorphism in linkage disequilibrium or genetic linkage with the gene polymorphism; and the probe set for determining a side effect that specifically hybridizes with the region amplified. The kit according to the present invention may comprise various reagents required for amplifying the above region, and/or various reagents required for specifically hybridizing the region amplified and the nucleic acid probe. The probe set for determining a side effect of trastuzumab can be immobilized to a carrier to prepare a DNA chip for determining a side effect of trastuzumab.

The present specification incorporates the disclosure of JP Patent Application No. 2019-41127A based on which the priority of the present application is claimed.

### Advantageous Effect of Invention

The present invention makes it possible to highly accurately determine a side effect from administration of trastuzumab by a simple means of detecting a gene polymorphism.

### Brief Description of Drawings

[Figure 1A] Figure 1A shows a regional association plot regarding a region containing rs9316695.
[Figure 1B] Figure 1B shows a regional association plot regarding a region containing rs28415722.
[Figure 1C] Figure 1C shows a regional association plot regarding a region containing rs7406710.
[Figure 1D] Figure 1D shows a regional association plot regarding a region containing rs11932853.
[Figure 1E] Figure 1E shows a regional association plot regarding a region containing rs8032978.
[Figure 2] Figure 2 is a graph showing ratios of patients developing trastuzumab-induced cardiotoxicity in a group of patients having a total score of 0 to 4 and a group of patients having a total score of 5 to 8 in which the total score was obtained per patient by the predictive scoring system constructed in Example.

### Description of Embodiments

The present invention relates to a method for determining a side effect from administration of trastuzumab based on the genotype of a gene polymorphism specified by one selected from the group consisting of rs9316695, rs11932853, rs28415722, rs7406710 and rs8032978, or a gene polymorphism in linkage disequilibrium or genetic linkage with the gene polymorphism. Trastuzumab (trade name: Herceptin) is a humanized monoclonal antibody targeting a human HER2 molecule and having a molecular weight of 148 kDa, and specifically binds to an epitope (aa529-625) in an extracellular region of the HER2 molecule. Trastuzumab is formulated together with additives, such as trehalose hydrate, L-histidine hydrochloride hydrate, L-histidine and polysorbate, into a composition for injection and used as a medicine. As a cancer which is observed to have overexpression of HER2, for example, breast cancer which is observed to have overexpression of HER2 and unresectable and progressive/recurrent stomach cancer which is observed to have overexpression of HER2, can be mentioned.

More specifically, according to the present invention, it is possible to determine side effects from administration of trastuzumab to various cancers mentioned above. Determining a side effect refers to determining the possibility of developing a side effect after administration of trastuzumab or determining the severity of the side effect. As a side effect of trastuzumab, at least one selected from heart failure, cardiogenic shock, pulmonary edema, pericardial effusion, cardiomyopathy, pericarditis, arrhythmia and bradycardia can be mentioned. In other words, a side effect can be defined as a symptom due to cardiotoxicity of trastuzumab.

For determining the genotype of a gene polymorphism, genomic DNA contained in a biological sample taken from a subject can be used. The biological sample taken from a subject herein is not particularly limited as long as it contains genomic DNA. Examples of the biological sample include blood and blood-related samples derived from blood (e.g., blood, serum and plasma); body fluids such as lymph, sweat, tears, saliva, urine, feces, ascites and cerebrospinal fluid; and crushed materials and extracts of cells, tissues or organs. A blood-related sample is preferably used in the present invention.

A means for extracting genomic DNA from a biological sample taken from a subject is not particularly limited, and a means of directly separating a DNA component from the biological sample, purifying and recovering it is preferred.

The gene polymorphism specified by rs9316695 (single nucleotide polymorphism, SNP) is located on the long arm of chromosome 13 (13q14.3) and has cytosine as wild-type and adenine as mutant. The gene polymorphism specified by rs1193853 (single nucleotide polymorphism, SNP) is located on the long arm of chromosome 4 (4q25) and is a single nucleotide polymorphism having thymine as wild-type and cytosine as mutant. The gene polymorphism specified by rs28415722 (single nucleotide polymorphism, SNP) is located on the long arm of chromosome 15 (15q26.3) and has guanine as wild-type and adenine as mutant. The gene polymorphism specified by rs7406710 (single nucleotide polymorphism, SNP) is located on the long arm of chromosome 17 (17q25.3) and has cytosine as wild-type and thymine as mutant. The gene polymorphism specified by rs8032978 (single nucleotide polymorphism, SNP) is located on the long arm of chromosome 15 (15q26.3) and has adenine as wild-type and guanine as mutant.

The term "linkage disequilibrium" refers to a population genetic phenomenon where a non-random correlation is observed among alleles of a plurality of loci or genetic markers (polymorphisms) in a biological population, more specifically, where a frequency of a specific combination (haplotype) of them significantly increases. The term "genetic linkage" refers to a genetic phenomenon where a combination of predetermined alleles is inherited from a parent to a child without following the Mendel's Law of Independent Assortment.

Specific examples of a gene polymorphism in linkage disequilibrium or genetic linkage with the gene polymorphism specified by rs9316695 include, but are not particularly limited to, rs4597194, rs67371330, rs9527155, rs73197793, rs9527156, rs9536600, rs9536601, rs9527157, rs9596894, rs9536604, rs9536605, rs9536606, rs9596895, rs12585722, rs9536608, rs9536610, rs4884826, rs147044674, rs201449129, rs199694348, rs146020011, rs9536611, rs9536612, rs67998663, rs9536613, rs9596896, rs9536614, rs144930433, rs144567553, rs9596897, rs4572266, rs7330060, rs9527161, rs4584708, rs4640062, rs140902703, rs143148342, rs17089212, rs2104970, rs9536598, rs9527160, rs4883836, rs150544413, rs11616925, rs9536595, rs7334767, rs59300548, rs9536609, rs4883837, rs11617903, rs7993293, rs9536620, rs9536619, rs58440048, rs10585368, rs9527169, rs529033940, rs17089149, rs1572184, rs2050281, rs12870784, rs2210644, rs9536584, rs9536586, rs9536588, rs9536589, rs4275742, rs78202205, rs67234964, rs145327528, rs12583122, rs17089167, rs4523820 and rs7994759 (r² > 0.4). Of them, a gene polymorphism in linkage disequilibrium or genetic linkage with the gene polymorphism specified by rs9316695 is preferably at least one gene polymorphism selected from the group consisting of rs4597194, rs67371330, rs9527155, rs73197793, rs9527156, rs9536600, rs9536601, rs9527157, rs9596894, rs9536604, rs9536605, rs9536606, rs9596895, rs12585722, rs9536608, rs9536610, rs4884826, rs147044674, rs201449129, rs199694348, rs146020011, rs9536611, rs9536612, rs67998663, rs9536613, rs9596896, rs9536614, rs144930433, rs144567553, rs9596897, rs4572266, rs7330060, rs9527161, rs4584708, rs4640062, rs140902703, rs143148342 and rs17089212 (r² = 1.000).

Specific examples of a gene polymorphism in linkage disequilibrium or genetic linkage with the gene polymorphism specified by rs11932853 include, but are not particularly limited to, rs13128178, rs13103305 and rs34290584 (r² > 0.8).

Specific examples of a gene polymorphism in linkage disequilibrium or genetic linkage with the gene polymorphism specified by rs28415722 include, but are not particularly limited to, rs28728168, rs11854776, rs1383149, rs4144489, rs13329373, rs12592103, rs2086366, rs12372962, rs28787308, rs4441250, rs11247348, rs1993976, rs1118043, rs2127556, rs8027435, rs202050468, rs28477300, rs11421357, rs12148125, rs12148342, rs28424020, rs28622146, rs28852783, rs74537059, rs4144488, rs12148124, rs1480097, rs60452357, rs28547243, rs200378270, rs28881820, rs28786836, rs79151393, rs76681857, rs62026165, rs62026166, rs67260482, rs67176041, rs113000837, rs112082590, rs79186877, rs12593815, rs67544351, rs66460935, rs28831185, rs112350672, rs9920073, rs28856352, rs9920491, rs9920100, rs899668, rs899670, rs899671, rs1600527, rs1842330, rs28886127, rs28763397, rs899669, rs2310786, rs2219877, rs28688638, rs66514354, rs28762186, rs5814843, rs113548268, rs28529334, rs28482307, rs28713196, rs28524985, rs1480096, rs7178508, rs2127555, rs7182053, rs8024637, rs4965928, rs4500701, rs899672, rs12148884, rs11247343, rs4305003, rs72756784, rs11857308, rs72756793, rs144244319, rs72756771, rs201638246, rs59400223, rs8041025, rs56254795, rs56087861, rs72756798, rs899673, rs7165184, rs2170105, rs190524788, rs145368286, rs72756742, rs58965013, rs2310788, rs200379847, rs12439514, rs9744177, rs4340314, rs7170047, rs145073022, rs59673774, rs1973203, rs7174886, rs4966026, rs6598551, rs7163138, rs11854601, rs899666, rs7171284, rs7169062, rs7171511, rs7178632, rs28476632, rs10660160, rs7163125, rs35915991, rs7169617, rs2871595, rs139776752, rs7169876, rs59784745, rs34967522, rs58591739, rs77673781, rs77476477, rs74848179, rs28804986, rs8026981, rs149769916, rs67999313, rs34527695, rs7172170, rs2089, rs72756785, rs62026191, rs76137345, rs112123401, rs7171292, rs7167815, rs28531668, rs4321178, rs72756790, rs2086365, rs201660876, rs55693761, rs56361500, rs28816992, rs59699060, rs62026167, rs28872593, rs4598889, rs4561450, rs4246339, rs78382254, rs28421057, rs28694303, rs4448919, rs28415561, rs12148823, rs7183126, rs11858646, rs62026209, rs111412942, rs62024083, rs113693221, rs12148449, rs12148742, rs62026207, rs62026208, rs201016517, rs11855844, rs4102909, rs11856483, rs28409382, rs59256589, rs62026196, rs58535173, rs142419518, rs34544919, rs62026205, rs56753756, rs79875962, rs12593369, rs11433091, rs11247363, rs11247364, rs11247366, rs12591387, rs12594533, rs12592382, rs8040797, rs8024782, rs62026194, rs149652096, rs113666132, rs8038239, rs145372032, rs74496658, rs11247360, rs66603848, rs4965371, rs56397226, rs76212696, rs28755859, rs28516952, rs9672462, rs9672677, rs113071929, rs12595170, rs369284370, rs7495211, rs751483, rs139593401, rs12439362, rs12438268, rs9672473, rs9672826, rs9806583, rs9806588, rs11247384, rs9672593, rs9330523, rs9672475, rs77969302, rs148140419, rs12441935, rs984998, rs72758712, rs139378984, rs12441957, rs12912787, rs72758714, rs984999, rs55978834, rs7179988, rs72758718, rs72758720, rs143988409, rs7180674, rs28671427, rs7163855, rs28368406 and rs34610227 (r² > 0.2). Of them, a gene polymorphism in linkage disequilibrium or genetic linkage with the gene polymorphism specified by rs28415722 is preferably at least one gene polymorphism selected from the group consisting of rs28728168, rs11854776, rs1383149, rs4144489, rs13329373, rs12592103, rs2086366, rs12372962, rs28787308, rs4441250, rs11247348, rs1993976, rs1118043, rs2127556 and rs8027435 (r² > 0.8). A gene polymorphism in linkage disequilibrium or genetic linkage with the gene polymorphism specified by rs28415722 is more preferably rs28728168 and/or rs11854776 (r² = 1.000).

Specific examples of a gene polymorphism in linkage disequilibrium or genetic linkage with the gene polymorphism specified by rs7406710 include, but are not particularly limited to, rs8081479, rs7406026, rs7405590, rs72854495, rs7405588, rs74530133, rs7405749, rs7406506, rs7405641, rs11552304, rs7405532, rs71675424, rs62076028, rs58483803, rs11869448, rs11870015, rs8074089, rs7405522, rs7224579, rs6565593, rs6565590, rs6565592, rs72854500, rs4076968, rs7213717, rs78537846, rs9675106, rs6565595, rs7207933, rs60016321, rs112791119, rs77387916, rs74818865, rs7207958, rs12453887, rs11150795, rs12675, rs61430049, rs112930265, rs6565591, rs8068081, rs6565596, rs8068511, rs3924327, rs61655749, rs34797307, rs35789723, rs2075722, rs7406756 and rs7406904 (r² > 0.2). Of them, a gene polymorphism in linkage disequilibrium or genetic linkage with the gene polymorphism specified by rs7406710 is preferably at least one gene polymorphism selected from the group consisting of rs8081479, rs7406026, rs7405590, rs72854495, rs7405588, rs74530133, rs7405749, rs7406506, rs7405641, rs11552304, rs7405532, rs71675424, rs62076028, rs58483803, rs11869448, rs11870015, rs8074089, rs7405522, rs7224579, rs6565593, rs6565590, rs6565592, rs72854500 and rs4076968 (r² > 0.4).

Specific examples of a gene polymorphism in linkage disequilibrium or genetic linkage with the gene polymorphism specified by rs8032978 include, but are not particularly limited to, rs8033540, rs8033003, rs28863221, rs28770217, rs111829181, rs55957523, rs28609156, rs28690028, rs28665122, rs7172856, rs143956992, rs117531330, rs77343149, rs74563564, rs149545605, rs11327127, rs117512970, rs74041962, rs59542966, rs2898864, rs4275835, rs111447514, rs61276520, rs60105028, rs75348190, rs1545855, rs74041979 and rs59199124 (r² > 0.6). Of them, a gene polymorphism in linkage disequilibrium or genetic linkage with the gene polymorphism specified by rs8032978 is preferably at least one gene polymorphism selected from the group consisting of rs8033540, rs8033003, rs28863221, rs28770217, rs111829181, rs55957523, rs28609156, rs28690028, rs28665122 and rs7172856 (r² > 0.8). A gene polymorphism in linkage disequilibrium or genetic linkage with the gene polymorphism specified by rs8032978 is more preferably rs8033540 (r² = 1.000).

As a method for specifying the genotype of a gene polymorphism mentioned above, more specifically, a method for typing a gene polymorphism, a method of analyzing a single nucleotide polymorphism known in the technical field can be used. Examples of the analysis method include a real time PCR method, a direct sequencing method, a TaqMan(R) PCR method, an invader(R) method, a Luminex(R) method, a quenching primer/probe (QP) method, MALDI-TOF method and a molecular beacon method. Specific examples of the method include a method comprising collecting a biological sample from a subject (usually meaning a human subject); amplifying a nucleic acid fragment containing a measurement target, a single nucleotide polymorphism site, by use of primers and in accordance with an amplification reaction using genomic DNA of the biological sample as a template; and detecting hybridization of the obtained nucleic acid fragment with a pair of probes corresponding to a wild-type and a mutant; or detecting a wild-type and a mutant using a specific probe to the single nucleotide polymorphism site in the above PCR amplification process.

A probe set for use in specifying a gene polymorphism (more specifically, a probe set for determining a side effect from administration of trastuzumab) may be any probe set as long as it contains an oligonucleotide that hybridizes, under stringent conditions, with a region of consecutive 5 to 50 nucleotides, preferably 10 to 40 nucleotides, more preferably 10 to 30 nucleotides containing a gene polymorphism specified by one selected from the group consisting of rs9316695, rs11932853, rs28415722, rs7406710 and rs8032978, or a gene polymorphism in linkage disequilibrium or genetic linkage with the gene polymorphism. If an oligo probe synthesized by using an artificial nucleic acid such as Locked Nucleic Acid (LNA) is used as a probe, the probe can specifically hybridize even with a short nucleotide. The probe set refers to a set of wild-type probe corresponding to a wild-type allele and a mutant probe corresponding to a mutant allele.

The stringent conditions refer to the conditions under which a specific hybrid is formed and a non-specific hybrid is not formed, and specific examples include conditions under which a hybrid can be formed in a solution containing 6 × SSC (a solution containing 1.5 M NaCl and 0.15 M trisodium citrate is 10 × SSC) and 50% formamide, at 45°C and then washed with 2 × SSC at 50°C. The stringent conditions can be set appropriately with reference to Molecular Biology, John Wiley & Sons, N. Y. (1989), 6.3.1-6.3.6. Alternatively, examples of the stringent conditions include the conditions under which a hybrid can be formed in a solution containing 3 × SSC/0.3 × SDS at 54°C and washed sequentially with cleaning liquid A (10 × SSC/1% SDS solution), cleaning liquid B (20 × SSC) and cleaning liquid C (5 × SSC) (see, JP Patent Publication (Kokai) No. 2011-250726 A).

A probe set for use in specifying a gene polymorphism may be immobilized on a carrier and used. Examples of the carrier include a planar substrate and spherical carrier like beads. Specific examples include a carrier described in JP Patent Publication (Kokai) No. 2011-250726A. A probe for detecting a wild-type and a probe for detecting a mutant may be immobilized to the same carrier or different carriers.

The primer for use in a method for specifying the above gene polymorphism may be a primer formed of an oligonucleotide that can amplify at least 5 consecutive nucleotides as a nucleic acid fragment containing a gene polymorphism specified by one selected from the group consisting of rs9316695, rs11932853, rs28415722, rs7406710 and rs8032978, or a gene polymorphism in linkage disequilibrium or genetic linkage with the gene polymorphism, using genomic DNA as a template. More specifically, a primer formed of an oligonucleotide that can amplify at least 5 nucleotides, preferably 10 to 500 nucleotides, more preferably 20 to 200 nucleotides, further preferably 50 to 100 nucleotides containing a gene polymorphism specified by one selected from the group consisting of rs9316695, rs11932853, rs28415722, rs7406710 and rs8032978, or a gene polymorphism in linkage disequilibrium or genetic linkage with the gene polymorphism, can be appropriately designed based on a genomic DNA sequence stored in a known database.

In amplifying at least 5 consecutive nucleotides containing a gene polymorphism specified by one selected from the group consisting of rs9316695, rs11932853, rs28415722, rs7406710 and rs8032978, or a gene polymorphism in linkage disequilibrium or genetic linkage with the gene polymorphism, the sequence amplified can be identified by use of a primer previously labeled or labeled nucleotides as substrates in an amplification reaction. Examples of the labeling substance include, but are not particularly limited to, a radioisotope, a fluorescent dye, and an organic compound such as digoxigenin (DIG) and biotin.

The probe or primer can be obtained through chemical synthesis, for example, by a nucleic acid synthesizer. Examples of the nucleic acid synthesizer that can be used include a DNA synthesizer and a full-automatic nucleic acid synthesizer.

If a nucleic acid fragment amplified has a label, a nucleic acid fragment hybridized with each probe (wild-type probe or mutant probe contained in a probe set for determining a side effect from administration of trastuzumab) can be measured by detecting the label. For example, if a fluorescent dye is used as a label, nucleic acid fragment hybridized with a probe can be measured by measuring the intensity of fluorescence emitted from the fluorescent dye. Specifically, when the ratio of a nucleic acid fragment hybridized with a wild-type probe and a nucleic acid fragment hybridized with a mutant probe, the ratio can be calculated from an output value when a label of a wild-type probe is detected and an output value when a label of a mutant probe is detected. If a fluorescent label is used as a label, fluorescence intensity is used as an output value.

More specifically, the value for determination can be obtained by dividing the output value (fluorescence intensity) derived from a nucleic acid fragment hybridized with a mutant probe by an average value of an output value (fluorescence intensity) derived from a nucleic acid fragment hybridized with a mutant and an output value (fluorescence intensity) derived from a nucleic acid fragment hybridized with a wild-type probe. The value for determination approximates to a normalized value of the amount of mutant contained in a nucleic acid fragment. In this manner, a single nucleotide polymorphism of a subject is analyzed based on the value for determination, and then, whether it is mutant homozygosity, wild-type homozygosity, or heterozygosity can be determined.

When the value for determination is used, in order to analyze a single nucleotide polymorphism of a subject and determine whether it is mutant homozygosity, wild-type homozygosity, or heterozygosity, it is preferable to previously set two thresholds different in level (threshold A and threshold B). Here, threshold A and threshold B herein are assumed to satisfy the relationship: threshold A > threshold B. More specifically, if the value for determination calculated as described above exceeds threshold A, it can be determined to be mutant homozygosity. If the value for determination is not more than threshold A and more than threshold B, it can be determined to be heterozygosity. If the value for determination is not more than threshold B, it can be determined to be wild-type homozygosity.

Examples of a method for setting threshold A and threshold B include, but are not particularly limited to, a method comprising calculating a value for determination by using a sample whose genotype is previously determined as described above, and calculating a probability density as normal distribution with respect to mutant homozygosity, wild-type homozygosity, or heterozygosity described above. In this case, an intersection (the position at which large and small probability density values switch and between maximum values of both probability densities) at which probability densities mutually overlap is obtained. Average values of each of mutant homozygosity, wild-type homozygosity, and heterozygosity described above, are obtained. The threshold of mutant homozygosity and heterozygosity can be calculated as an average value of (average value of mutant homozygosity and average value of heterozygosity) and an average value of the intersection. Similarly, the threshold of heterozygosity and wild-type homozygosity can be calculated as an average value of (average value of heterozygosity and average value of wild-type homozygosity) and an average value of the intersection.

In the gene polymorphism specified by rs9316695, a mutant (adenine) is a risk allele. If the gene polymorphism specified by rs9316695 in a subject is wild-type (cytosine) homozygosity, it is determined that the subject has a low possibility of developing a side effect of trastuzumab. If the gene polymorphism is mutant/wild-type heterozygosity, it is determined that the subject has a high possibility of developing a side effect of trastuzumab. If the gene polymorphism is mutant homozygosity, it is determined that the subject has a higher possibility of developing a side effect of trastuzumab.

In the gene polymorphism specified by rs11932853, a wild-type (thymine) is a risk allele. If the gene polymorphism specified by rs11932853 in a subject is mutant (cytosine) homozygosity, it is determined that the subject has a low possibility of developing a side effect of trastuzumab. If the gene polymorphism is mutant/wild-type heterozygosity, it is determined that the subject has a high possibility of developing a side effect of trastuzumab. If the gene polymorphism is wild-type homozygosity, it is determined that the subject has a higher possibility of developing a side effect of trastuzumab.

In the gene polymorphism specified by rs28415722, a mutant (adenine) is a risk allele. If the gene polymorphism specified by rs28415722 in a subject is wild-type (guanine) homozygosity, it is determined that the subject has a low possibility of developing a side effect of trastuzumab. If the gene polymorphism is mutant/wild-type heterozygosity, it is determined that the subject has a low possibility of developing a side effect of trastuzumab. If the gene polymorphism is mutant homozygosity, it is determined that the subject has a higher possibility of developing a side effect of trastuzumab.

In the gene polymorphism specified by rs7406710, a wild-type (cytosine) is a risk allele. If the gene polymorphism specified by rs7406710 in a subject is mutant (thymine) homozygosity, it is determined that the subject has a low possibility of developing a side effect of trastuzumab. If the gene polymorphism is mutant/wild-type heterozygosity, it is determined that the subject has a low possibility of developing a side effect of trastuzumab. If the gene polymorphism is wild-type homozygosity, it is determined that the subject has a higher possibility of developing a side effect of trastuzumab.

In the gene polymorphism specified by rs8032978, a wild-type (guanine) is a risk allele. If the gene polymorphism specified by rs8032978 in a subject is wild-type (adenine) homozygosity, it is determined that the subject has a low possibility of developing a side effect of trastuzumab. If the gene polymorphism is mutant/wild-type heterozygosity, it is determined that the subject has a high possibility of developing a side effect of trastuzumab. If the gene polymorphism is mutant homozygosity, it is determined that the subject has a higher possibility of developing a side effect of trastuzumab.

A subject for which determination is made is, for example, a person suspected or diagnosed to have a disease within the application range of trastuzumab mentioned above and is not particularly limited.

In determining a side effect of trastuzumab, one gene polymorphism selected from a gene polymorphism specified by one selected from the group consisting of rs9316695, rs11932853, rs28415722, rs7406710 and rs8032978, or a gene polymorphism in linkage disequilibrium or genetic linkage with the gene polymorphism as mentioned above may be used or a plurality of gene polymorphisms may be used in combination. In other words, a side effect of trastuzumab in a subject can be determined based on the genotype of one gene polymorphism selected from a gene polymorphism specified by one selected from the group consisting of rs9316695, rs11932853, rs28415722, rs7406710 and rs8032978, or a gene polymorphism in linkage disequilibrium or genetic linkage with the gene polymorphism as mentioned above. Alternatively, a side effect of trastuzumab in a subject can be determined based on a plurality of genotypes of gene polymorphisms selected from these gene polymorphisms.

As a plurality of the gene polymorphisms, for example, among five types of gene polymorphisms specified by rs9316695, rs11932853, rs28415722, rs7406710 and rs8032978, two types, three types, four types or five types of gene polymorphisms can be arbitrarily put in use. At this time, the genotype of each gene polymorphism is scored and a side effect of trastuzumab of a subject may be determined based on the score. For example, a score can be given such that the score becomes high if the possibility of developing a side effect of trastuzumab is high, whereas the score becomes low if the possibility of developing a side effect of trastuzumab is low. In contrast, a score can be given such that the score becomes low if the possibility of developing a side effect of trastuzumab is high, whereas the score becomes high if the possibility of developing a side effect of trastuzumab is low.

To describe scoring more specifically, a score of 0 can be given if the gene polymorphism specified by rs9316695 is wild-type (cytosine) homozygosity, since the possibility of developing a side effect of trastuzumab can be determined to be low. A score of 1 can be given if the gene polymorphism is mutant /wild-type heterozygosity, since the possibility of developing a side effect of trastuzumab can be determined to be high. A score of 2 can be given if the gene polymorphism is mutant homozygosity, since the possibility of developing a side effect of trastuzumab can be determined to be higher. Similarly, a score of 0 can be given if the gene polymorphism specified by rs11932853 is mutant (cytosine) homozygosity, since the possibility of developing a side effect of trastuzumab can be determined to be low. A score of 1 can be given if the gene polymorphism is mutant/wild-type heterozygosity, since the possibility of developing a side effect of trastuzumab can be determined to be high. A score of 2 can be given if the gene polymorphism is mutant homozygosity, since the possibility of developing a side effect of trastuzumab can be determined to be higher. Similarly, a score of 0 can be given if the gene polymorphism specified by rs28415722 is wild-type (guanine) homozygosity, since the possibility of developing a side effect of trastuzumab can be determined to be low. A score of 0 can be given if the gene polymorphism is mutant /wild-type heterozygosity, since the possibility of developing a side effect of trastuzumab can be determined to be low. A score of 2 can be given if the gene polymorphism is mutant homozygosity, since the possibility of developing a side effect of trastuzumab can be determined to be higher. Similarly, a score of 0 can be given if the gene polymorphism specified by rs7406710 is mutant (thymine) homozygosity, since the possibility of developing a side effect of trastuzumab can be determined to be low. A score of 0 can be given if the gene polymorphism is mutant/wild-type heterozygosity, since the possibility of developing a side effect of trastuzumab can be determined to be low. A score of 2 can be given if the gene polymorphism is wild-type homozygosity, since the possibility of developing a side effect of trastuzumab can be determined to be higher. Similarly, a score of 0 can be given if the gene polymorphism specified by rs8032978 is wild-type (adenine) homozygosity, since the possibility of developing a side effect of trastuzumab can be determined to be is low. A score of 1 can be given if the gene polymorphism is mutant/wild-type heterozygosity, since the possibility of developing a side effect of trastuzumab can be determined to be high. A score of 2 can be given if the gene polymorphism is mutant homozygosity, since the possibility of developing a side effect of trastuzumab is determined to be higher.

As described above, with respect to the five types of gene polymorphisms specified by rs9316695, rs11932853, rs28415722, rs7406710 and rs8032978, a score in the range of 0 to 2 is given depending on the genotypes. In this manner, the genotypes of these five types of gene polymorphisms in a subject can be scored. In the above cases, it can be determined that the higher the score, the higher the possibility of developing a side effect of trastuzumab.

To be more specific, a score in the range of 0 to 2 is given depending on the genotypes with respect to the five types of gene polymorphisms specified by rs9316695, rs11932853, rs28415722, rs7406710 and rs8032978 as mentioned above, and the total of scores given to the genotypes determined in a subject falls within the range of 0 to 10. The total score of a subject can be used as a reference data for predicting the possibility of developing a side effect of trastuzumab in the subject. For example, if a subject has a total score of 5 or more, the subject can be determined to belong to a group having a high possibility of developing a side effect of trastuzumab. Conversely, if a subject has a total score of is 4 or less, the subject can be determined to belong to a group having a low possibility of developing a side effect of trastuzumab.

In the above example, a total score of 5 or more (4 or less) is used as the reference for determining whether or not a subject belongs to a group having a high possibility of developing a side effect of trastuzumab; however, the reference can be a total score of 6 or more, 7 or more, or 8 or more. The threshold of the total score may be arbitrarily determined.

### Example

Now, the present invention will be more specifically described by way of Example. The technical scope of the present invention is not limited to that of the following Example.

### [Materials and methods]

### [Patients]

In this Example, the samples of 268 patients treated with trastuzumab were selected from the samples registered at the NCC Biobank during the period from February 2010 to December 2015, and subjected to a genome-wide association study (GWAS) for identifying genetic markers related to a risk of trastuzumab-induced cardiotoxicity. In a follow-up study, 213 patients who received a treatment with trastuzumab (14 cases and 199 controls) during the period from October 2017 to March 2018 were collected from the hospital of St. Marianna University Hospital and Nakagami Hospital; and collected from the samples registered at the NCC Biobank during the period from June 2016 to October 2017.

In this Example, in accordance with the standard of the Herceptin adjuvant (HERA) test, a case exhibiting a left ventricular ejection fraction (LVEF) of less than 45% after administration of trastuzumab or a case having a decrease of 10% or more from a baseline and exhibiting an LVEF of less than 50% was defined as a case of trastuzumab-induced cardiotoxicity. This Example was approved by the ethics committees of the National Cancer Center Japan, St. Marianna University School of Medicine, Nakagami Hospital and the Japanese Foundation For Cancer Research. Informed consent was obtained from all patients participated in this Example.

### [Genotype determination and quality control]

Genomic DNA was extracted from cancer cell-free peripheral blood or formalin fixative paraffin embedded (FFPE) lymph node. The genotypes of 268 patients were determined by using Infinium OmniExpressExome-8 v1.4 (manufactured by Illumina, Inc.) for GWAS analysis. In this Example, quality control (call rate: 99% or more) was applied to both of cases and controls. To the controls, the Hardy-Weinberg equilibrium (P > 1.0 × 10⁻⁶) was applied and a minor allele frequency of SNP was set at 0.01 or more. 543,807 SNPs on the autosomal chromosomes passed quality control. In this Example, potential relevance of the samples was evaluated by using the identity-by-state (IBS) method.

Structure of a population was confirmed by the principal component analysis (PCA) using EIGENSTRAT software 6.0.1. Distribution of a sample population was determined by PCA in comparison with three reference populations from 1,000 Genomes Project Phase 3 database. The 1,000 Genomes Project Phase 3 database contains data of Europeans (represented by CEPH from Utah (CEU)), Africans (represented by Yoruba in Ibadan (YRI)) and east Asians (represented by Japanese in Tokyo (JPT), Han Chinese in Beijing (CHB), southern Han Chinese (CHS), Chinese Dai in Xishuangbanna (CDX) and Kinh in Ho Chi Minh City, Vietnam (KHV)). PCA was carried out based on the genotype information from the samples used in this Example. Fisher's exact test for the P value predicted was carried out and a quantile-quantile plot (Q-Q plot) was prepared between the P values determined by the test. As a result, a genomic inflation factor was 1.173 and a significant stratification of a population was not shown (not shown).

### [Genotype imputation]

Genome-wide imputation of 268 patients used in GWAS was performed. 43 SNPs located in three genomic regions (locus in chromosome 13q14.3 and independent two loci in chromosome 15q26.3) exhibited a significance greater than marker SNP (tag-SNP) in individual loci by genome-wide imputation analysis, and thus, these genomic regions (54593774-54618139 of chromosome 13q14.3, 98578726-98646496 of chromosome 15q26.3 and 101796748-101800094 of chromosome 15q26.3) in 213 patients used in a follow-up study were also subjected to imputation analysis.

In this Example, a reference panel based on 1,000 Genomes Project Phase 3 integrated release version 5 regarding east Asian individuals including Japanese in Tokyo, Chinese in Beijing and Chinese in South China, was used for imputation. Genotype data in which genotypes were not determined by Minimac 3 software were subjected to imputation. Variants showing RSQ > 0.3, which is a threshold of imputation quality, were extracted. In the imputation analysis in this Example, SNP quality control was carried out by removing SNPs having a genotype present at a low rate of less than 99% and deviated from a Hardy-Weinberg equilibrium (P ≤ 1.0 × 10⁻⁶) and SNPs having a minor allele frequency of < 0.01 in the control.

### [Statistical analysis]

In the GWAS analysis and follow-up study, a case-control referent study based on the Fisher's exact test was applied to three genetic models, i.e., allele model, dominant genetic model and recessive genetic model. A non-risk allele or a non-risk genotype was used as a reference, and the odds ratio (OR) and confidence interval (CI) were calculated with respect to one of these three genetic models having the lowest P value. For the multiple test for these three genetic models, the genome-wide significance level after the Bonferroni correction was P = 3.06 × 10⁻⁸[0.05/(543,807 × 3)] in GWAS and P = 1.67 × 10⁻⁴[0.05/(100 × 3)] in the follow-up study.

For combination analysis, the number of persons in each genotype in the follow-up study was added to those in GWAS. The age distribution, primary lesion, whether a pretreatment with anthracycline was applied or not, and statuses of an HER2 receptor and a hormone receptor were evaluated by logistic regression analysis as to whether they become risk factors for trastuzumab-induced cardiotoxicity.

In the scoring system for predicting a risk of trastuzumab-induced cardiotoxicity, a score of 2 was given to an individual having homozygous risk alleles, a score of 1 was given to an individual having heterozygous risk alleles, and a score of 0 was given to an individual having homozygous non-risk alleles with respect to gene polymorphisms of rs9316695, rs11932853 and rs8032978, in this Example. In this Example, with respect to the genotypes of rs28415722 and rs7406710, a score of 2 was given to an individual having homozygous risk alleles and a score of 0 was given to an individual having other genotypes. In this Example, the scores given to individual gene polymorphisms were added up and the total score was obtained per individual. Owing to the scoring system, individual patients were classified into 9 groups (total score: 0, 1, 2, 3, 4, 5, 6, 7 or 8).

In this Example, all statistical analyses were carried out by R statistical environment version 3.3.1, PLINK version 1.07 or BellCurve for Excel (manufactured by Social Survey Research Information Co., Ltd.). Regional association plots were prepared by use of Locus Zoom.

### [Results]

### [Background factor of patient]

To identify genetic markers for determining a risk of trastuzumab-induced cardiotoxicity, 481 patients who received a treatment with trastuzumab were employed in this Example. The 481 patients includes 25 cases (having trastuzumab-induced cardiotoxicity) and 456 controls (having no trastuzumab-induced cardiotoxicity). In Table 1, the background factors of these 481 patients were collectively shown.

**[Table 1]**

| | | GWAS (N=268) | | Follow-up study (N=213) | | Total number (N=481) | |
|---|---|---|---|---|---|---|---|
| Patient's background factor | | Number of patients (%) | | Number of patients (%) | | Number of patients (%) | |
| | | Case (N=11) | Control (N=257) | Case (N=14) | Control (N=199) | Case (N=25) | Control (N=456) |
| Age (Age) | | | | | | | |
| | Median value | 62.0 | 61.0 | 60.0 | 57.0 | 62.0 | 59.0 |
| | Range | 46-76 | 29-86 | 36-81 | 27-83 | 36-81 | 27-86 |

| Primary lesion | | | | | | | |
|---|---|---|---|---|---|---|---|
| | Breast cancer | 10 (90.9) | 185 (72.0) | 14 (100.0) | 178 (89.4) | 24 (96.0) | 363 (79.6) |
| | Stomach cancer | 1 (9.1) | 68 (26.5) | 0 (0.0) | 21 (10.6) | 1 (4.0) | 89 (19.5) |
| | Unknown | 0 (0.0) | 4 (1.6) | 0 (0.0) | 0 (0.0) | 0 (0.0) | 4 (0.9) |

| Pretreatment with anthracycline | | | | | | | |
|---|---|---|---|---|---|---|---|
| | Treated | 9 (81.8) | 148 (57.6) | 10 (71.4) | 128 (64.3) | 19 (76.0) | 276 (60.5) |
| | Not treated | 2 (18.2) | 109 (42.4) | 4 (28.6) | 71 (35.7) | 6 (24.0) | 180 (39.5) |

| Her-2 | | | | | | | |
|---|---|---|---|---|---|---|---|
| | Positive | 10 (90.9) | 249 (96.9) | 14 (100.0) | 199 (100.0) | 24 (96.0) | 448 (98.2) |
| | Negative | 0 (0.0) | 0 (0.0) | 0 (0.0) | 0 (0.0) | 0 (0.0) | 0 (0.0) |
| | Unknown | 1 (9.1) | 8 (3.1) | 0 (0.0) | 0 (0.0) | 1 (4.0) | 8 (1.8) |

| ER status (breast cancer) | | | | | | | |
|---|---|---|---|---|---|---|---|
| | Positive | 6 (60.0) | 119 (64.3) | 11 (78.6) | 117 (65.7) | 17 (70.8) | 236 (65.0) |
| | Negative | 3 (30.0) | 64 (34.6) | 3 (21.4) | 61 (34.3) | 6 (25.0) | 125 (34.4) |
| | Unknown | 1 (10.0) | 2 (1.1) | 0 (0.0) | 0 (0.0) | 1 (4.2) | 2 (0.6) |

| PR status (breast cancer) | | | | | | | |
|---|---|---|---|---|---|---|---|
| | Positive | 6 (60.0) | 104 (56.2) | 8 (57.1) | 92 (51.7) | 14 (58.3) | 196 (54.0) |
| | Negative | 4 (40.0) | 80 (43.2) | 6 (42.9) | 86 (48.3) | 10 (41.7) | 166 (45.7) |
| | Unknown | 0 (0.0) | 1 (0.5) | 0 (0.0) | 0 (0.0) | 0 (0.0) | 1 (0.3) |

| | | | | | | | |
|---|---|---|---|---|---|---|---|
| ER: estrogen receptor, PR: progesterone receptor | | | | | | | |

In both of the cases and controls, the median age of the patients in the beginning of a treatment with trastuzumab was 56. The background factors of patients listed in Table 1 did not show a significant relationship with trastuzumab-induced cardiotoxicity in logistic regression analysis.

### [GWAS and follow-up study analysis]

In this Example, trastuzumab-induced cardiotoxicity was defined as a case exhibiting a left ventricular ejection fraction (LVEF) of less than 45% after administration of trastuzumab or a case having a decrease of 10% or more from a baseline and exhibiting an LVEF of less than 50%, in accordance with the standard of the Herceptin adjuvant (HERA) test. GWAS of 268 patients was carried out by using Infinium OmniExpressExome-8 v1.4 (manufactured by Illumina, Inc.).

After general quality control, the association study of 543,807 SNPs was carried out by the Fisher's exact test based on three genetic models (allele model, dominant genetic model and recessive genetic model). In this Example, a quantile-quantile plot (Q-Q plot) was prepared. The genomic inflation factor was 1.173, which means that there is no group stratification. However, an SNP reaching the genome-wide significance level was not found. In GWAS, SNPs ranked in the top 100 significant difference showed a possibility of being associated with trastuzumab-induced cardiotoxicity (P = 7.60 × 10⁻⁷ to 2.01 × 10⁻⁴). To further check the results of GWAS, independent 213 patients including 14 cases and 199 control cases were subjected to a follow-up study on these 100 SNPs. In the follow-up study, there were no SNPs reached a significance level after the Bonferroni correction; however, 17 SNPs which may be associated with cardiotoxicity were identified (P = 3.6 × 10⁻² to 9.4 × 10⁻²). As a result of combination analysis of GWAS and the follow-up study of these 17 SNPs, 9 SNPs were found in 5 loci exhibiting stronger association than that in GWAS results. These 9 SNPs were rs9316695 (P_{combined} = 6.00 × 10⁻⁶), rs9527156 (P_{combined} = 8.64 × 10⁻⁶), rs12583122 (P_{combined} = 1.92 × 10⁻⁵) and rs11617903 (P_{combined} = 2.22 × 10⁻⁵) located on chromosome 13q14.3; rs11932853 (P_{combined} = 1.42 × 10⁻⁴) located on chromosome 4q25; rs1383149 (P_{combined} = 1.01 × 10⁻⁴) and rs12372962 (P_{combined} = 1.15 × 10⁻⁴) located on chromosome 15q26.3; rs7406710 (P_{combined} = 1.07 × 10⁻⁴) located on chromosome 17q25.3; and rs8032978 (P_{combined} = 1.60 × 10^{- 4}) located on chromosome 15q26.3. None of them, however, satisfied the genome-wide significance level (P = 3.06 × 10⁻⁸).

### [Imputation analysis]

To further identify candidate loci associated with trastuzumab-induced cardiotoxicity in this Example, genome wide imputation analysis of GWAS samples was performed to check association. However, new candidate loci associated with trastuzumab-induced cardiotoxicity were not identified. From the five candidate loci (a single locus on chromosome 4q25, a single locus on chromosome 13q14.3, two independent loci on chromosome 15q26.3, and a single locus on chromosome 17q25.3) identified by GWAS, 43 SNPs were newly identified in three genetic regions (a single locus on chromosome 13q14.3 and two independent loci on chromosome 15q26.3) by imputation analysis. These SNPs showed more significant association than the marker SNP (tag-SNP) identified in GWAS.

In this Example, a follow-up study was further carried out using 213 independent patients with respect to these 43 SNPs. As a result, SNP having strong association was not further found other than marker SNPs in these three loci. However, when GWAS results and follow-up study results were used in combination with respect to these 43 SNPs, it was found that rs28415722 on chromosome 15q26.3 is more strongly associated with trastuzumab-induced cardiotoxicity than in GWAS. In addition, rs28415722 showed the strongest association in this region (P_{combined} = 8.88 × 10⁻⁵).

Information, analysis results and statistical analysis results of 5 SNPs associated with trastuzumab-induced cardiotoxicity and identified in this Example were collectively shown in Table 2.

**[Table 2]**

| | | | | | | | Case | | | | Control | | | | | P value | | |
|---|---|---|---|---|---|---|---|---|---|---|---|---|---|---|---|---|---|---|
| Chromosomal region | Location^{a} | SNP | Allele ^{b} [1/2] | Risk allele | Analysis stage | Genotyping/ Imputation | Number of persons by genotype | | | RAF | Number of persons by genotype | | | RAF | Allele model | Dominant genetic model | Recessive genetic model | Odds ratio (95% Cl)^{c} |
| | | | | | | | 11 | 12 | 22 | | 11 | 12 | 22 | | | | | |
| 13q14.3 | 54,615,773 | rs9316695 | C/A | A | GWAS/ follow-up study combination | Genotyped | 3 | 5 | 3 | 0.50 | 199 | 55 | 3 | 0.12 | 2.59 × 10⁻⁵ | 8.22 × 10⁻⁴ | 9.71 × 10⁴ | 7.38 (2.77-19.66) |
| | | | | | | | 7 | 6 | 1 | 0.29 | 153 | 43 | 3 | 0.12 | 3.79 × 10⁻² | 4.80 × 10⁻² | 2.40 × 10⁻¹ | 2.84 (1.02-7.19) |
| | | | | | | | 10 | 11 | 4 | 0.38 | 352 | 98 | 6 | 0.12 | 6.00 × 10-⁶ | 1.22 × 10⁻⁴ | 9.73 × 10⁻⁴ | 4.46 (2.30-8.47) |
| 15q26.3 | 98,609,746 | ^{d}rs28415722 | G/A | A | GWAS/ follow-up study combination | Imputed | 1 | 2 | 8 | 0.82 | 95 | 119 | 43 | 0.40 | 1.97 × 10⁻⁴ | 1.04 × 10⁻¹ | 1.10 × 10⁻⁴ | 13.08(2.99-79.58) |
| | | | | | | | 6 | 3 | 5 | 0.46 | 82 | 85 | 32 | 0.37 | 4.21 × 10⁻¹ | 1.00 | 7.31 × 10⁻² | 2.88(0.71-10.34) |
| | | | | | | | 7 | 5 | 13 | 0.62 | 177 | 204 | 75 | 0.39 | 1.62 × 10⁻³ | 3.98 × 10⁻¹ | 8.88 × 10⁻⁵ | 5.48(2.21-13.69) |
| 17q25.3 | 79,505,624 | rs7406710 | C/T | C | GWAS/ follow-up study combination | Genotyped | 11 | 0 | 0 | 1.00 | 111 | 119 | 27 | 0.66 | 2.38 × 10⁻⁴ | 6.10 × 10⁻¹ | 1.35 × 10⁻⁴ | NA |
| | | | | | | | 10 | 3 | 1 | 0.82 | 90 | 93 | 16 | 0.69 | 2.01 × 10⁻¹ | 1.00 | 9.37 × 10⁻² | 3.01 (0.83-13.61) |
| | | | | | | | 21 | 3 | 1 | 0.90 | 201 | 212 | 43 | 0.67 | 4.55 × 10⁴ | 7.17 × 10⁻¹ | 1.07 × 10⁻⁴ | 6.64 (2.19-27.01) |
| 4q25 | 112,024,388 | rs11932853 | T/C | T | GWAS/ follow-up study combination | Genotyped | 7 | 3 | 1 | 0.77 | 31 | 129 | 97 | 0.37 | 2.26 × 10⁻⁴ | 6.04 × 10⁻² | 1.47 × 10⁻⁴ | 12.54(2.99-61.88) |
| | | | | | | | 4 | 8 | 2 | 0.57 | 27 | 99 | 73 | 0.38 | 7.05 × 10⁻² | 1.45 × 10⁻¹ | 1.28 × 10⁻¹ | 2.13 (0.92-5.08) |
| | | | | | | | 11 | 11 | 3 | 0.66 | 58 | 228 | 170 | 0.38 | 1.42 × 10⁻⁴ | 9.56 × 10⁻³ | 2.10 × 10⁻⁴ | 3.20(1.70-6.23) |
| 15q26.3 | 101,799,790 | rs8032978 | A/G | G | GWAS/ follow-up study combination | Genotyped | 6 | 4 | 1 | 0.27 | 239 | 18 | 0 | 0.04 | 1.95 × 10⁻⁴ | 9.84 × 10⁻⁴ | 4.10 × 10⁻² | 10.22 (2.93-31.90) |
| | | | | | | | 11 | 3 | 0 | 0.11 | 185 | 13 | 1 | 0.04 | 1.07 × 10⁻¹ | 8.87 × 10⁻² | 1.00 | 3.57 (0.57-15.86) |
| | | | | | | | 17 | 7 | 1 | 0.18 | 424 | 31 | 1 | 0.04 | 1.60 × 10⁻⁴ | 4.29 × 10⁻⁴ | 1.01 × 10⁻¹ | 5.83 (2.30-13.51) |

| | | | | | | | | | | | | | | | | | | |
|---|---|---|---|---|---|---|---|---|---|---|---|---|---|---|---|---|---|---|
| ^{a}: based on GRCh37 genome assembly ^{b}: reference allele (GRCh37) is defined as Allele 1 ^{c}: odds ratio of model having minimum P value is shown ^{d}: genotype rs28415722 is confirmed by using TaqMan SNP genotyping assay GWAS: genomic wide association study, SNP: single nucleotide polymorphism, RAF: risk allele frequency, Cl: confidence interval, NA: no data | | | | | | | | | | | | | | | | | | |

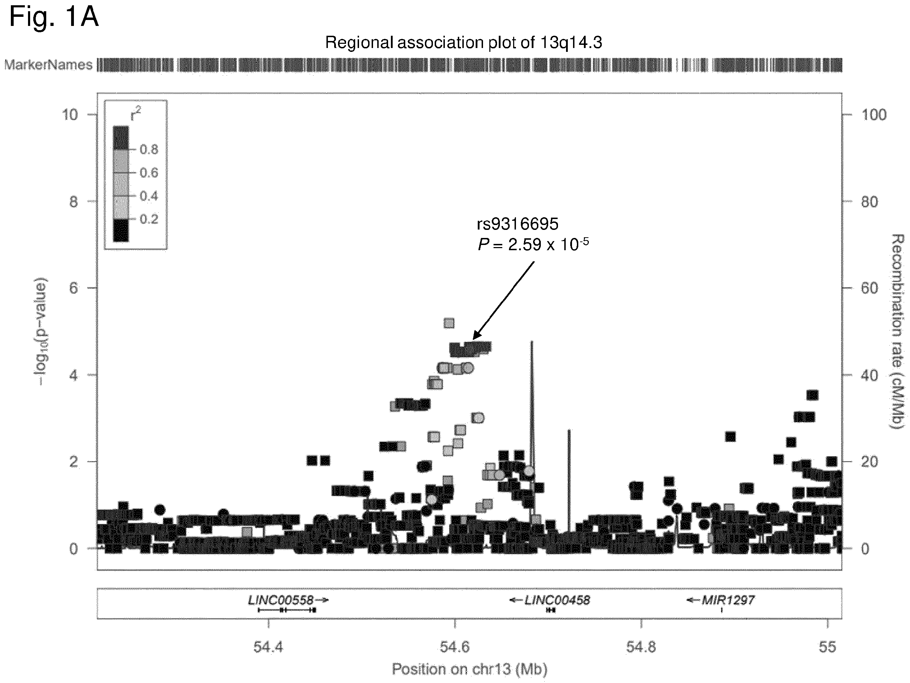

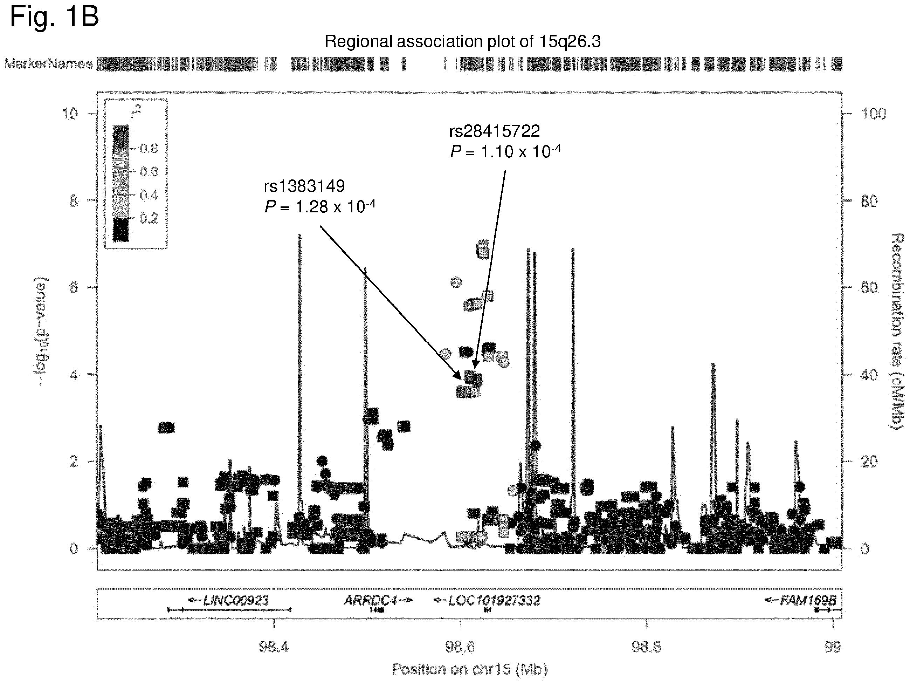

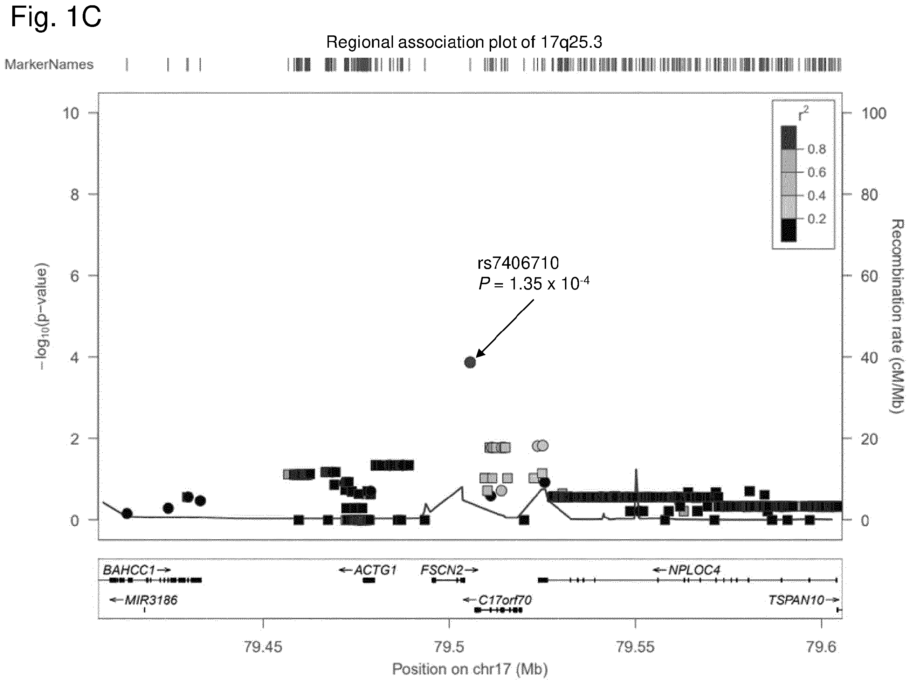

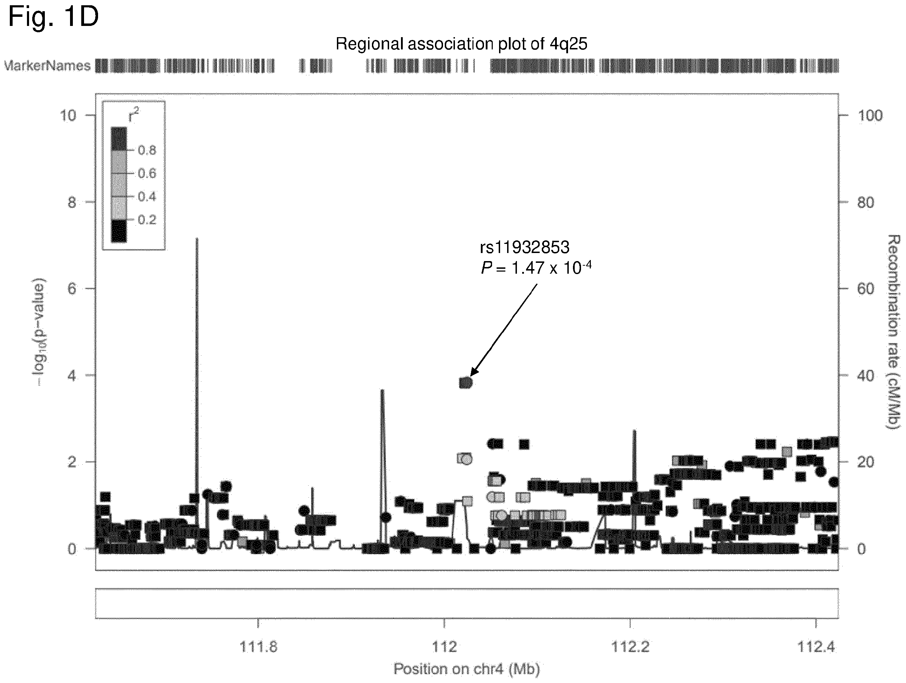

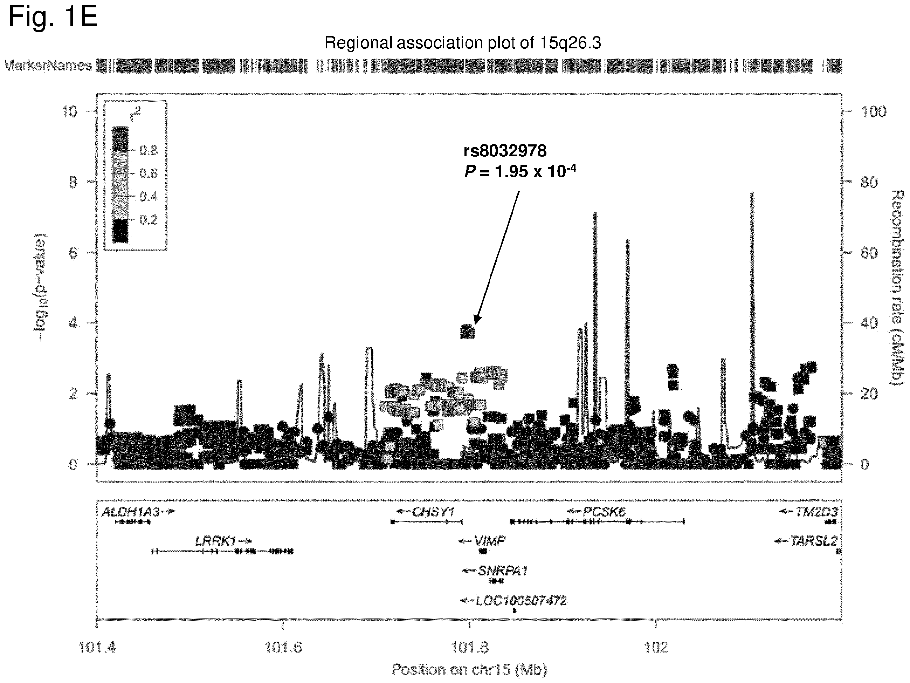

Regional association plots of these 5 SNPs to the regions containing respective SNPs were prepared and shown in Figures 1A to E. Note that, Figure 1A shows a regional association plot regarding a region containing rs9316695; Figure 1B a regional association plot regarding a region containing rs28415722; Figure 1C a regional association plot regarding a region containing rs7406710; Figure 1D a regional association plot regarding a region containing rs11932853; and Figure 1E a regional association plot regarding a region containing rs8032978.

In Figures 1A to E, the P values (-log₁₀ (P value)) of SNPs genotyped are plotted by circles, whereas the P values (-log₁₀ (P value)) of SNPs imputed are plotted by squares. In Figures 1A to E, the horizontal axis represents physical positions on the chromosome. In Figures 1A to E, genetic recombination rates estimated from 1000 genome samples of Japanese (JPT) in Tokyo and Han Chinese (CHB) in Beijing are shown by lines. Contrasting densities of individual plots represent linkage disequilibrium (LD) on the scale of r² = 0 to 1. In the region containing SNPs pointed by arrows in the figures, SNPs close to r² = 1.0 are densely present. The lower stages of regional association plots of Figures 1A to E show gene annotations, which are available at the genome browser developed by the University of California, Santa Cruz.

### [Predictive scoring system]

In this Example, a predictive scoring system for evaluating trastuzumab-induced cardiotoxicity was constructed using 5 SNPs identified as described above. The 5 SNPs (rs9316695, rs28415722, rs7406710, rs11932853 and rs8032978), which showed the lowest P value in the combination analysis, were regarded as independent predictive factors for trastuzumab-induced cardiotoxicity by logistic regression analysis. Accordingly, genotypes of these 5 SNPs were used in combination to construct a scoring system in this Example.

The predictive scoring system is a system in which scores are given to individual patients in consideration of the genotypes and the number of risk alleles.

To describe more specifically, with respect to rs28415722 and rs7406710, a score of 2 was given to an individual having homozygous risk alleles, whereas a score of 0 was given to an individual having other genotypes. This is because these rs28415722 and rs7406710 showed the lowest P value in recessive genetic models.

In contrast, with respect to rs9316695, rs 11932853 and rs8032978, a score of 2 was given to an individual having homozygous risk alleles; a score of 1 was given to an individual having heterozygous risk alleles; and a score of 0 was given to an induvial having homozygous non-risk alleles. In this Example, the scores given to individual gene polymorphisms were added up and the total score was obtained per individual. The results are shown in Table 3.

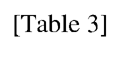

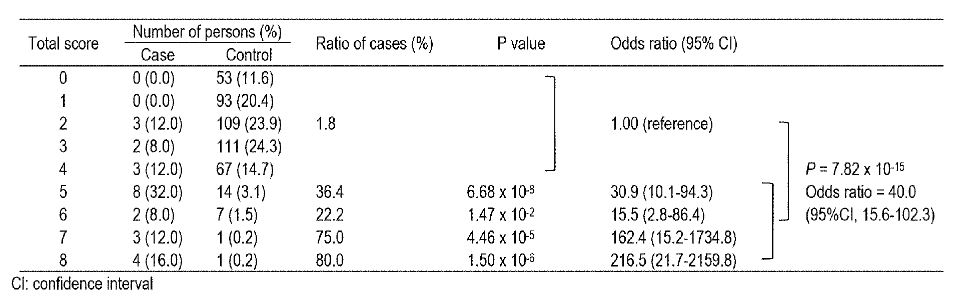

As shown in Tabel 3, the predictive scording system constructed in this Example made it possible to classify patients into 9 groups (total score: 0,1,2,3,4,5,6,7 or 8). There was a tendency that the higher the score for prediction, the higher the ratio of patients developing trastuzumab-induced cardiotoxicity. More specifically, in the group having a total score of 0 to 4, the ratio of cases (ratio of patients developing trastuzumab-induced cardiotoxicity) was 1.8% (8/441). This ratio was 36.4% (8/22) in the group having a total score of 5, 22.2% (2/9) in the group having a total score of 6, 75.0% (3/4) in the group having a total score of 7, and 80.0% (4/5) in the group having a total score of 8.

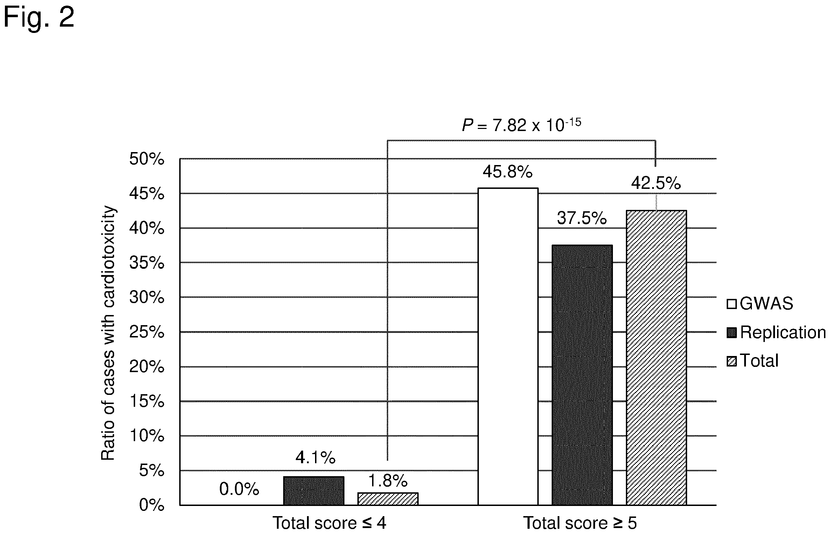

In this Example, in consideration of the sensitivity (68.0%) and specificity (95.0%) of the predictive scoring system, study was conducted by dividing the patients into two groups, i.e., a group having a total score of 0 to 4 and a group having a total score of 5 to 8. As a result, as shown in Figure 2, the incidence rates of trastuzumab-induced cardiotoxicity in the group having a total score of 5 to 8 were 45.8% (11/24), 37.5% (6/16) and 42.5% (17/40) in GWAS, follow-up study and combination analysis thereof, respectively, whereas the incidence rates were 0% (0/244), 4.1% (8/197) and 1.8% (8/441) in the group having a total score of 0 to 4. This demonstrates that onset of trastuzumab-induced cardiotoxicity can be effectively predicted before starting a treatment with trastuzumab according to the predictive scoring system using the 5 SNPs mentioned above (P_{combined} = 7.82 × 10⁻¹⁵, odds ratio = 40.0, 95% confidence interval = 15.6 to 102.3).

### [Gene polymorphism in genetic linkage]

In this Example, a gene polymorphism in linkage disequilibrium or genetic linkage was searched with respect to each of the 5 SNPs (rs9316695, rs28415722, rs7406710, rs11932853 and rs8032978). More specifically, data of 1000 Genomes Project were searched to find a gene polymorphism satisfying the conditions that r² is a predetermined value or more with respect to each SNP and a minor allele frequency (MAF) is a predetermined value or more (≤ 0.01). The gene polymorphism was defined as a gene polymorphism in linkage disequilibrium or genetic linkage with the SNP. As the gene polymorphism satisfying r² ≥ 0.4 with respect to rs9316695, 74 gene polymorphisms shown in Table 4 were identified.

**[Table 4]**

| SNP | Location ^{a} | Type | Reference allele ^{a} | Alternative allele ^{a} | Minor allele | Minor allele frequency | r² |
|---|---|---|---|---|---|---|---|
| rs4597194 | 54599654 | SNP | C | T | T | 0.116 | 1.000 |
| rs67371330 | 54600359 | SNP | G | A | A | 0.116 | 1.000 |
| rs9527155 | 54601173 | SNP | G | A | A | 0.116 | 1.000 |
| rs73197793 | 54603016 | SNP | A | G | G | 0.116 | 1.000 |
| rs9527156 | 54603479 | SNP | C | T | T | 0.116 | 1.000 |
| rs9536600 | 54603549 | SNP | T | C | C | 0.116 | 1.000 |
| rs9536601 | 54604128 | SNP | T | G | G | 0.116 | 1.000 |
| rs9527157 | 54604157 | SNP | G | C | C | 0.116 | 1.000 |
| rs9596894 | 54604722 | SNP | A | C | C | 0.116 | 1.000 |
| rs9536604 | 54607416 | SNP | T | C | C | 0.116 | 1.000 |
| rs9536605 | 54607479 | SNP | G | C | C | 0.116 | 1.000 |
| rs9536606 | 54607518 | SNP | C | T | T | 0.116 | 1.000 |
| rs9596895 | 54609719 | SNP | A | G | G | 0.116 | 1.000 |
| rs12585722 | 54610042 | SNP | T | C | C | 0.116 | 1.000 |
| rs9536608 | 54611679 | SNP | C | T | T | 0.116 | 1.000 |
| rs9536610 | 54611850 | SNP | G | A | A | 0.116 | 1.000 |
| rs4884826 | 54612274 | SNP | T | C | C | 0.116 | 1.000 |
| rs147044674 | 54612330 | SNP | C | T | T | 0.116 | 1.000 |
| rs201449129 | 54612795 | Indel | AC | A | A | 0.116 | 1.000 |
| rs199694348 | 54612803 | Indel | TG | T | T | 0.116 | 1.000 |
| rs146020011 | 54613130 | SNP | T | C | C | 0.116 | 1.000 |
| rs9536611 | 54613563 | SNP | A | G | G | 0.116 | 1.000 |
| rs9536612 | 54613571 | SNP | A | C | C | 0.116 | 1.000 |
| rs67998663 | 54613871 | SNP | A | G | G | 0.116 | 1.000 |
| rs9536613 | 54614847 | SNP | A | T | T | 0.116 | 1.000 |
| rs9596896 | 54614866 | SNP | G | C | C | 0.116 | 1.000 |
| rs9536614 | 54615113 | SNP | A | T | T | 0.116 | 1.000 |
| rs144930433 | 54615315 | Indel | CA | C | C | 0.116 | 1.000 |
| rs144567553 | 54615571 | Indel | ATAGAT | A | A | 0.116 | 1.000 |
| rs9596897 | 54615729 | SNP | C | T | T | 0.116 | 1.000 |
| rs4572266 | 54616772 | SNP | G | C | C | 0.116 | 1.000 |
| rs7330060 | 54618139 | SNP | G | A | A | 0.116 | 1.000 |
| rs9527161 | 54618390 | SNP | C | T | T | 0.116 | 1.000 |
| rs4584708 | 54620094 | SNP | T | A | A | 0.116 | 1.000 |
| rs4640062 | 54621749 | SNP | T | C | C | 0.116 | 1.000 |
| rs140902703 | 54622772 | SNP | G | A | A | 0.116 | 1.000 |
| rs143148342 | 54624548 | Indel | ACCTATAGTC | A | A | 0.116 | 1.000 |
| rs17089212 | 54633571 | SNP | C | T | T | 0.116 | 1.000 |
| rs2104970 | 54603212 | SNP | G | C | C | 0.115 | 0.990 |
| rs9536598 | 54595870 | SNP | T | C | C | 0.116 | 0.981 |
| rs9527160 | 54613415 | SNP | C | T | T | 0.119 | 0.972 |
| rs4883836 | 54611988 | SNP | C | T | T | 0.15 | 0.745 |
| rs150544413 | 54631666 | Indel | A | AAATAATAAT | AAATAATAAT | 0.085 | 0.710 |
| rs11616925 | 54593774 | SNP | G | C | C | 0.134 | 0.686 |
| rs9536595 | 54595054 | SNP | A | G | G | 0.134 | 0.686 |
| rs7334767 | 54570654 | SNP | T | C | C | 0.137 | 0.652 |
| rs59300548 | 54603014 | SNP | A | G | G | 0.171 | 0.638 |
| rs9536609 | 54611716 | SNP | T | G | G | 0.171 | 0.638 |
| rs4883837 | 54612146 | SNP | T | C | C | 0.171 | 0.638 |
| rs11617903 | 54614352 | SNP | A | G | G | 0.171 | 0.638 |
| rs7993293 | 54637297 | SNP | G | A | A | 0.076 | 0.630 |
| rs9536620 | 54639898 | SNP | A | G | G | 0.076 | 0.630 |
| rs9536619 | 54634578 | SNP | C | A | A | 0.075 | 0.621 |
| rs58440048 | 54645035 | Indel | AAAAC | AAAACAAAC | AAAACAAAC | 0.075 | 0.621 |
| rs10585368 | 54646030 | Indel | GACA | G | G | 0.075 | 0.621 |
| rs9527169 | 54647817 | SNP | A | G | G | 0.075 | 0.621 |
| rs529033940 | 54644705 | SNP | G | A | A | 0.074 | 0.612 |
| rs17089149 | 54577597 | SNP | C | A | A | 0.163 | 0.511 |
| rs1572184 | 54603148 | SNP | G | T | G | 0.216 | 0.476 |
| rs2050281 | 54605052 | SNP | G | A | G | 0.216 | 0.476 |
| rs12870784 | 54605940 | SNP | G | A | G | 0.216 | 0.476 |
| rs2210644 | 54571858 | SNP | G | C | C | 0.182 | 0.454 |
| rs9536584 | 54575712 | SNP | G | A | A | 0.18 | 0.449 |
| rs9536586 | 54577963 | SNP | G | A | A | 0.18 | 0.449 |
| rs9536588 | 54579343 | SNP | C | T | T | 0.18 | 0.449 |
| rs9536589 | 54579358 | SNP | G | A | A | 0.18 | 0.449 |
| rs4275742 | 54581241 | SNP | G | C | C | 0.18 | 0.449 |
| rs78202205 | 54616146 | SNP | T | C | C | 0.056 | 0.448 |
| rs67234964 | 54611555 | Indel | C | CAA | C | 0.227 | 0.447 |
| rs145327528 | 54591861 | Indel | G | GGGAA | GGGAA | 0.196 | 0.419 |
| rs12583122 | 54586240 | SNP | G | A | A | 0.199 | 0.410 |
| rs17089167 | 54586840 | SNP | T | G | G | 0.199 | 0.410 |
| rs4523820 | 54587973 | SNP | G | A | A | 0.199 | 0.410 |
| rs7994759 | 54588151 | SNP | T | C | C | 0.199 | 0.410 |

| | | | | | | | |
|---|---|---|---|---|---|---|---|
| ^{a}: based on GRCh37 genome assembly SNP: single nucleotide polymorphism, Indel (insertion/deletion): insertion/deletion, r²: linkage disequilibrium coefficient | | | | | | | |

As the gene polymorphism satisfying r² ≥ 0.2 with respect to rs28415722, 248 gene polymorphisms shown in Table 5 were identified.

**[Table 5]**

| SNP | Location ^{a} | Type | Reference allele ^{a} | Alternative allele ^{a} | Minor allele | Minor allele frequency | r² |
|---|---|---|---|---|---|---|---|
| rs28728168 | 98611986 | SNP | G | A | A | 0.387 | 1.000 |
| rs11854776 | 98613684 | SNP | G | A | A | 0.387 | 1.000 |
| rs1383149 | 98610131 | SNP | A | G | G | 0.388 | 0.996 |
| rs4144489 | 98614923 | SNP | T | C | C | 0.389 | 0.992 |
| rs13329373 | 98614824 | SNP | G | A | A | 0.390 | 0.988 |
| rs12592103 | 98616662 | SNP | T | C | C | 0.390 | 0.988 |
| rs2086366 | 98617241 | SNP | C | T | T | 0.389 | 0.983 |
| rs12372962 | 98617828 | SNP | T | C | C | 0.392 | 0.979 |
| rs28787308 | 98599510 | SNP | G | A | A | 0.391 | 0.975 |
| rs4441250 | 98602241 | SNP | C | T | T | 0.391 | 0.975 |
| rs11247348 | 98602847 | SNP | G | A | A | 0.391 | 0.975 |
| rs1993976 | 98606313 | SNP | G | A | A | 0.396 | 0.955 |
| rs1118043 | 98605348 | SNP | C | T | T | 0.397 | 0.951 |
| rs2127556 | 98605613 | SNP | A | T | T | 0.397 | 0.951 |
| rs8027435 | 98624886 | SNP | A | G | G | 0.378 | 0.889 |
| rs202050468 | 98605113 | Indel | CTTTTT | C | C | 0.330 | 0.625 |
| rs28477300 | 98596649 | SNP | C | T | C | 0.478 | 0.578 |
| rs11421357 | 98611315 | Indel | C | CA | C | 0.477 | 0.576 |
| rs12148125 | 98608746 | SNP | T | C | T | 0.476 | 0.574 |
| rs12148342 | 98608818 | SNP | A | G | A | 0.476 | 0.574 |
| rs28424020 | 98615147 | SNP | C | T | C | 0.476 | 0.574 |
| rs28622146 | 98596697 | SNP | G | A | G | 0.475 | 0.571 |
| rs28852783 | 98597574 | SNP | C | T | C | 0.475 | 0.571 |
| rs74537059 | 98598676 | SNP | T | C | T | 0.475 | 0.571 |
| rs4144488 | 98608281 | SNP | G | A | G | 0.475 | 0.571 |
| rs12148124 | 98608693 | SNP | T | C | T | 0.475 | 0.571 |
| rs1480097 | 98611412 | SNP | C | G | C | 0.478 | 0.571 |
| rs60452357 | 98596812 | Indel | C | CA | C | 0.474 | 0.569 |
| rs28547243 | 98596943 | SNP | T | C | T | 0.474 | 0.569 |
| rs200378270 | 98597464 | Indel | ATC | A | ATC | 0.474 | 0.569 |
| rs28881820 | 98597509 | SNP | A | T | A | 0.474 | 0.569 |
| rs28786836 | 98597612 | SNP | T | C | T | 0.474 | 0.569 |
| rs79151393 | 98598194 | Indel | A | AAT | A | 0.474 | 0.569 |
| rs76681857 | 98598290 | Indel | A | AT | A | 0.474 | 0.569 |
| rs62026165 | 98598400 | SNP | T | C | T | 0.474 | 0.569 |
| rs62026166 | 98598403 | SNP | C | T | C | 0.474 | 0.569 |
| rs67260482 | 98598534 | SNP | G | T | G | 0.474 | 0.569 |
| rs67176041 | 98598634 | SNP | A | G | A | 0.474 | 0.569 |
| rs113000837 | 98598684 | Indel | TAC | T | TAC | 0.474 | 0.569 |
| rs112082590 | 98617116 | Indel | G | GT | G | 0.474 | 0.569 |
| rs79186877 | 98617119 | SNP | C | A | C | 0.474 | 0.569 |
| rs12593815 | 98596529 | SNP | C | T | C | 0.473 | 0.567 |
| rs67544351 | 98598602 | SNP | C | G | C | 0.473 | 0.567 |
| rs66460935 | 98598641 | SNP | A | G | A | 0.473 | 0.567 |
| rs28831185 | 98599246 | SNP | T | G | T | 0.473 | 0.567 |
| rs112350672 | 98599529 | Indel | TA | T | TA | 0.473 | 0.567 |
| rs9920073 | 98599660 | SNP | T | C | T | 0.473 | 0.567 |
| rs28856352 | 98599686 | SNP | G | A | G | 0.473 | 0.567 |
| rs9920491 | 98599761 | SNP | C | T | C | 0.473 | 0.567 |
| rs9920100 | 98599946 | SNP | T | C | T | 0.473 | 0.567 |
| rs899668 | 98601467 | SNP | G | A | G | 0.473 | 0.567 |
| rs899670 | 98601779 | SNP | T | C | T | 0.473 | 0.567 |
| rs899671 | 98601846 | SNP | G | T | G | 0.473 | 0.567 |
| rs1600527 | 98604458 | SNP | C | T | C | 0.473 | 0.567 |
| rs1842330 | 98604647 | SNP | A | G | A | 0.473 | 0.567 |
| rs28886127 | 98599288 | SNP | C | T | C | 0.472 | 0.565 |
| rs28763397 | 98599289 | SNP | A | G | A | 0.472 | 0.565 |
| rs899669 | 98601721 | SNP | C | T | C | 0.472 | 0.565 |
| rs2310786 | 98602642 | SNP | T | C | T | 0.472 | 0.565 |
| rs2219877 | 98604007 | SNP | A | G | A | 0.472 | 0.565 |
| rs28688638 | 98620941 | SNP | T | C | T | 0.472 | 0.565 |
| rs66514354 | 98598500 | SNP | C | G | C | 0.475 | 0.564 |
| rs28762186 | 98599355 | SNP | G | T | G | 0.475 | 0.564 |
| rs5814843 | 98601416 | Indel | G | GT | G | 0.475 | 0.564 |
| rs113548268 | 98600941 | Indel | C | CTTTTGTTTT GTTT | C | 0.477 | 0.562 |
| rs28529334 | 98597229 | SNP | C | A | C | 0.468 | 0.556 |
| rs28482307 | 98597252 | SNP | C | T | C | 0.468 | 0.556 |
| rs28713196 | 98597269 | SNP | A | T | A | 0.468 | 0.556 |
| rs28524985 | 98597280 | SNP | A | T | A | 0.468 | 0.556 |
| rs1480096 | 98605991 | SNP | C | T | C | 0.468 | 0.556 |
| rs7178508 | 98606157 | SNP | C | G | C | 0.467 | 0.554 |
| rs2127555 | 98605496 | SNP | C | A | C | 0.466 | 0.551 |
| rs7182053 | 98623689 | SNP | A | G | A | 0.487 | 0.507 |
| rs8024637 | 98624500 | SNP | C | T | C | 0.487 | 0.507 |
| rs4965928 | 98595970 | SNP | G | C | G | 0.475 | 0.480 |
| rs4500701 | 98588488 | SNP | G | A | G | 0.489 | 0.444 |
| rs899672 | 98627683 | SNP | T | G | T | 0.477 | 0.438 |
| rs12148884 | 98601045 | SNP | A | T | T | 0.218 | 0.435 |
| rs11247343 | 98597809 | SNP | T | C | C | 0.407 | 0.433 |
| rs4305003 | 98600728 | SNP | A | G | G | 0.217 | 0.432 |
| rs72756784 | 98611504 | SNP | C | G | G | 0.213 | 0.430 |
| rs11857308 | 98615560 | SNP | C | T | T | 0.216 | 0.429 |
| rs72756793 | 98617306 | SNP | T | C | C | 0.216 | 0.429 |
| rs144244319 | 98599378 | Indel | A | AC | AC | 0.215 | 0.427 |
| rs72756771 | 98604999 | SNP | C | G | G | 0.215 | 0.427 |
| rs201638246 | 98605130 | SNP | T | A | A | 0.220 | 0.424 |
| rs59400223 | 98615970 | SNP | G | T | T | 0.213 | 0.422 |
| rs8041025 | 98624655 | SNP | G | C | C | 0.214 | 0.417 |
| rs56254795 | 98620203 | SNP | A | C | C | 0.216 | 0.414 |
| rs56087861 | 98620322 | SNP | G | C | C | 0.216 | 0.414 |
| rs72756798 | 98623290 | SNP | A | T | T | 0.215 | 0.411 |
| rs899673 | 98627699 | SNP | G | A | G | 0.487 | 0.401 |
| rs7165184 | 98584277 | SNP | C | T | T | 0.486 | 0.399 |
| rs2170105 | 98629534 | SNP | C | T | C | 0.486 | 0.399 |
| rs190524788 | 98545559 | SNP | T | C | T | 0.336 | 0.388 |
| rs145368286 | 98546400 | SNP | C | T | C | 0.335 | 0.385 |
| rs72756742 | 98584928 | SNP | C | A | A | 0.219 | 0.383 |
| rs58965013 | 98589858 | SNP | G | A | A | 0.218 | 0.381 |
| rs2310788 | 98631862 | SNP | A | G | A | 0.486 | 0.380 |
| rs200379847 | 98649579 | SNP | C | T | C | 0.490 | 0.372 |
| rs12439514 | 98649075 | SNP | G | A | G | 0.491 | 0.370 |
| rs9744177 | 98649281 | SNP | C | T | C | 0.492 | 0.368 |
| rs4340314 | 98647382 | SNP | C | T | C | 0.489 | 0.362 |
| rs7170047 | 98640071 | SNP | C | A | A | 0.481 | 0.356 |
| rs145073022 | 98638357 | Indel | T | TG | TG | 0.492 | 0.355 |
| rs59673774 | 98640488 | SNP | C | T | T | 0.495 | 0.355 |
| rs1973203 | 98646197 | SNP | G | T | G | 0.498 | 0.355 |
| rs7174886 | 98636405 | SNP | T | C | C | 0.498 | 0.354 |
| rs4966026 | 98633358 | SNP | C | T | T | 0.493 | 0.353 |
| rs6598551 | 98635858 | SNP | A | G | G | 0.496 | 0.353 |
| rs7163138 | 98636442 | SNP | A | C | C | 0.496 | 0.353 |
| rs11854601 | 98643174 | SNP | C | G | G | 0.496 | 0.353 |
| rs899666 | 98646537 | SNP | C | T | C | 0.499 | 0.352 |
| rs7171284 | 98646791 | SNP | A | G | G | 0.497 | 0.351 |
| rs7169062 | 98646809 | SNP | C | G | G | 0.497 | 0.351 |
| rs7171511 | 98646948 | SNP | A | G | G | 0.497 | 0.351 |
| rs7178632 | 98650798 | SNP | G | A | G | 0.494 | 0.351 |
| rs28476632 | 98582877 | SNP | C | G | C | 0.497 | 0.349 |
| rs10660160 | 98634213 | Indel | C | CAG | CAG | 0.495 | 0.349 |
| rs7163125 | 98634919 | SNP | T | C | C | 0.495 | 0.349 |
| rs35915991 | 98642510 | Indel | AT | A | A | 0.481 | 0.347 |
| rs7169617 | 98644429 | SNP | T | C | C | 0.493 | 0.347 |
| rs2871595 | 98627997 | SNP | T | C | C | 0.224 | 0.345 |
| rs139776752 | 98636835 | SNP | G | C | C | 0.491 | 0.345 |
| rs7169876 | 98637621 | SNP | A | G | G | 0.491 | 0.345 |
| rs59784745 | 98642860 | SNP | T | C | C | 0.491 | 0.345 |
| rs34967522 | 98583771 | Indel | GT | G | GT | 0.488 | 0.342 |
| rs58591739 | 98646312 | SNP | C | A | A | 0.491 | 0.340 |
| rs77673781 | 98626738 | SNP | G | A | A | 0.207 | 0.339 |
| rs77476477 | 98647486 | SNP | G | A | A | 0.207 | 0.339 |
| rs74848179 | 98639180 | SNP | C | T | T | 0.489 | 0.338 |
| rs28804986 | 98565780 | SNP | G | A | G | 0.497 | 0.337 |
| rs8026981 | 98574201 | SNP | T | C | T | 0.495 | 0.335 |
| rs149769916 | 98637469 | SNP | T | A | A | 0.485 | 0.335 |
| rs67999313 | 98578726 | Indel | A | AT | A | 0.496 | 0.333 |
| rs34527695 | 98559566 | Indel | GC | G | GC | 0.496 | 0.332 |
| rs7172170 | 98583617 | SNP | C | T | T | 0.497 | 0.331 |
| rs2089 | 98610817 | SNP | G | A | A | 0.173 | 0.331 |
| rs72756785 | 98611915 | SNP | G | A | A | 0.173 | 0.331 |
| rs62026191 | 98612063 | SNP | G | A | A | 0.173 | 0.331 |
| rs76137345 | 98614251 | Indel | C | CT | CT | 0.173 | 0.331 |
| rs112123401 | 98561969 | Indel | TCTACCTCCC CCAG | T | TCTACCTCC CCCAG | 0.498 | 0.329 |
| rs7171292 | 98563397 | SNP | T | C | T | 0.498 | 0.329 |
| rs7167815 | 98563790 | SNP | C | T | C | 0.498 | 0.329 |
| rs28531668 | 98561379 | SNP | C | T | C | 0.497 | 0.325 |
| rs4321178 | 98659387 | SNP | T | C | T | 0.322 | 0.324 |
| rs72756790 | 98616390 | SNP | C | T | T | 0.173 | 0.323 |
| rs2086365 | 98617199 | SNP | C | T | T | 0.173 | 0.323 |
| rs201660876 | 98627210 | Indel | GAAA | G | G | 0.215 | 0.323 |
| rs55693761 | 98629345 | SNP | C | A | A | 0.215 | 0.323 |
| rs56361500 | 98629629 | SNP | A | G | G | 0.215 | 0.323 |
| rs28816992 | 98618123 | SNP | C | A | A | 0.172 | 0.320 |
| rs59699060 | 98631446 | Indel | CGAGA | C | C | 0.216 | 0.319 |
| rs62026167 | 98608618 | SNP | C | A | A | 0.171 | 0.318 |
| rs28872593 | 98597569 | SNP | T | C | C | 0.174 | 0.317 |
| rs4598889 | 98560885 | SNP | A | C | A | 0.496 | 0.316 |
| rs4561450 | 98569809 | SNP | G | T | G | 0.213 | 0.310 |
| rs4246339 | 98595622 | SNP | G | A | A | 0.176 | 0.307 |
| rs78382254 | 98599285 | SNP | A | G | G | 0.176 | 0.307 |
| rs28421057 | 98576914 | SNP | T | A | T | 0.208 | 0.305 |
| rs28694303 | 98577730 | SNP | T | C | T | 0.208 | 0.305 |
| rs4448919 | 98663026 | SNP | G | A | G | 0.334 | 0.305 |
| rs28415561 | 98576763 | SNP | A | G | A | 0.212 | 0.300 |
| rs12148823 | 98646562 | SNP | C | G | G | 0.216 | 0.298 |
| rs7183126 | 98646911 | SNP | T | C | C | 0.216 | 0.298 |
| rs11858646 | 98647987 | SNP | G | C | C | 0.216 | 0.298 |
| rs62026209 | 98646295 | SNP | G | A | A | 0.215 | 0.296 |
| rs111412942 | 98647450 | SNP | G | A | A | 0.215 | 0.296 |
| rs62024083 | 98648336 | SNP | C | T | T | 0.215 | 0.295 |
| rs113693221 | 98647460 | SNP | G | T | T | 0.217 | 0.294 |
| rs12148449 | 98646502 | SNP | A | T | T | 0.214 | 0.293 |
| rs12148742 | 98646806 | SNP | G | A | A | 0.211 | 0.293 |
| rs62026207 | 98645572 | SNP | G | A | A | 0.216 | 0.292 |
| rs62026208 | 98646061 | SNP | G | A | A | 0.213 | 0.291 |
| rs201016517 | 98581250 | Indel | CTT | C | CTT | 0.195 | 0.288 |
| rs11855844 | 98645222 | SNP | G | A | A | 0.212 | 0.288 |
| rs4102909 | 98650803 | SNP | C | T | C | 0.228 | 0.287 |
| rs11856483 | 98651114 | SNP | G | T | T | 0.217 | 0.287 |
| rs28409382 | 98561487 | SNP | A | G | A | 0.211 | 0.284 |
| rs59256589 | 98634901 | SNP | C | T | T | 0.209 | 0.281 |
| rs62026196 | 98634373 | SNP | C | T | T | 0.208 | 0.279 |
| rs58535173 | 98634730 | SNP | G | A | A | 0.208 | 0.279 |
| rs142419518 | 98638029 | SNP | A | G | G | 0.208 | 0.279 |
| rs34544919 | 98641256 | SNP | G | C | C | 0.208 | 0.279 |
| rs62026205 | 98644144 | SNP | G | A | A | 0.208 | 0.279 |
| rs56753756 | 98644443 | SNP | G | A | A | 0.208 | 0.279 |
| rs79875962 | 98605534 | SNP | G | A | A | 0.148 | 0.275 |
| rs12593369 | 98624180 | SNP | T | C | C | 0.162 | 0.275 |
| rs11433091 | 98625993 | Indel | C | CG | CG | 0.165 | 0.274 |
| rs11247363 | 98623942 | SNP | C | T | T | 0.164 | 0.272 |
| rs11247364 | 98623984 | SNP | A | T | T | 0.164 | 0.272 |
| rs11247366 | 98624159 | SNP | T | A | A | 0.164 | 0.272 |
| rs12591387 | 98624251 | SNP | G | T | T | 0.164 | 0.272 |
| rs12594533 | 98624254 | SNP | A | G | G | 0.164 | 0.272 |
| rs12592382 | 98624268 | SNP | C | A | A | 0.164 | 0.272 |
| rs8040797 | 98624483 | SNP | G | C | C | 0.164 | 0.272 |
| rs8024782 | 98624605 | SNP | C | T | T | 0.164 | 0.272 |
| rs62026194 | 98633560 | SNP | G | A | A | 0.205 | 0.271 |
| rs149652096 | 98637626 | SNP | G | T | T | 0.205 | 0.271 |
| rs113666132 | 98624365 | Indel | T | TTGAA | TTGAA | 0.163 | 0.270 |
| rs8038239 | 98624981 | SNP | T | C | C | 0.163 | 0.270 |
| rs145372032 | 98625370 | Indel | ATAAT | A | A | 0.163 | 0.270 |
| rs74496658 | 98612031 | SNP | G | A | A | 0.143 | 0.264 |
| rs11247360 | 98622378 | SNP | A | G | G | 0.160 | 0.263 |
| rs66603848 | 98578345 | Indel | C | CT | CT | 0.406 | 0.259 |
| rs4965371 | 98590218 | SNP | C | T | T | 0.149 | 0.254 |
| rs56397226 | 98647293 | SNP | T | A | A | 0.227 | 0.253 |
| rs76212696 | 98627021 | Indel | CTG | C | C | 0.167 | 0.243 |
| rs28755859 | 98558204 | SNP | C | A | C | 0.218 | 0.236 |
| rs28516952 | 98558208 | SNP | C | A | C | 0.218 | 0.236 |
| rs9672462 | 98558116 | SNP | G | A | G | 0.217 | 0.233 |
| rs9672677 | 98558480 | SNP | A | C | A | 0.217 | 0.233 |
| rs113071929 | 98581799 | Indel | AT | A | AT | 0.268 | 0.233 |
| rs12595170 | 98656293 | SNP | T | C | T | 0.477 | 0.233 |
| rs369284370 | 98559520 | Indel | TTCTC | T | TTCTC | 0.222 | 0.232 |
| rs7495211 | 98559756 | SNP | C | T | C | 0.223 | 0.228 |
| rs751483 | 98628640 | SNP | A | G | G | 0.157 | 0.228 |
| rs139593401 | 98629687 | Indel | AGGGAGGAG GAGGAAGAG GGAAAG | A | A | 0.157 | 0.228 |
| rs12439362 | 98655739 | SNP | G | C | G | 0.462 | 0.227 |
| rs12438268 | 98661409 | SNP | G | C | G | 0.462 | 0.227 |
| rs9672473 | 98558115 | SNP | A | T | A | 0.214 | 0.226 |
| rs9672826 | 98558426 | SNP | T | C | T | 0.214 | 0.226 |
| rs9806583 | 98558944 | SNP | T | C | T | 0.222 | 0.226 |
| rs9806588 | 98559015 | SNP | T | C | T | 0.222 | 0.226 |
| rs11247384 | 98655981 | SNP | G | A | G | 0.460 | 0.225 |
| rs9672593 | 98558065 | SNP | T | C | T | 0.213 | 0.224 |
| rs9330523 | 98558068 | SNP | A | G | A | 0.213 | 0.224 |
| rs9672475 | 98558197 | SNP | A | C | A | 0.241 | 0.224 |
| rs77969302 | 98559410 | Indel | CAGG | C | CAGG | 0.224 | 0.224 |
| rs148140419 | 98629463 | Indel | AAAAAAT | A | A | 0.158 | 0.223 |
| rs12441935 | 98661552 | SNP | A | T | A | 0.459 | 0.222 |
| rs984998 | 98661994 | SNP | G | A | G | 0.459 | 0.222 |
| rs72758712 | 98630299 | SNP | A | G | G | 0.154 | 0.221 |
| rs139378984 | 98642497 | Indel | G | GT | GT | 0.173 | 0.220 |
| rs12441957 | 98661587 | SNP | A | G | A | 0.460 | 0.220 |
| rs12912787 | 98559120 | SNP | T | C | C | 0.219 | 0.219 |
| rs72758714 | 98631507 | SNP | G | A | A | 0.153 | 0.219 |
| rs984999 | 98662309 | SNP | A | C | A | 0.457 | 0.216 |
| rs55978834 | 98653915 | SNP | A | G | G | 0.169 | 0.214 |
| rs7179988 | 98638305 | SNP | G | C | C | 0.150 | 0.212 |
| rs72758718 | 98641355 | SNP | T | C | C | 0.150 | 0.212 |
| rs72758720 | 98642095 | SNP | T | C | C | 0.150 | 0.212 |
| rs143988409 | 98643399 | Indel | CATTAT | C | C | 0.153 | 0.212 |
| rs7180674 | 98644635 | SNP | A | G | G | 0.150 | 0.212 |
| rs28671427 | 98583904 | SNP | T | C | C | 0.160 | 0.206 |
| rs7163855 | 98646496 | SNP | G | A | A | 0.144 | 0.206 |
| rs28368406 | 98583916 | SNP | T | C | C | 0.158 | 0.202 |
| rs34610227 | 98625340 | Indel | C | CA | CA | 0.124 | 0.201 |

| | | | | | | | |
|---|---|---|---|---|---|---|---|
| ^{a}: based on GRCh37 genome assembly SNP: single nucleotide polymorphism, Indel (insertion/deletion): insertion/deletion, r²: linkage disequilibrium coefficient | | | | | | | |

As the gene polymorphism satisfying r² ≥ 0.2 with respect to rs7406710, 50 gene polymorphisms shown in Table 6 were identified.

**[Table 6]**

| SNP | Location ^{a} | Type | Reference allele ^{a} | Alternative allele ^{a} | Minor allele | Minor allele frequency | *r²* |
|---|---|---|---|---|---|---|---|
| rs8081479 | 79509589 | SNP | C | G | G | 0.118 | 0.463 |
| rs7406026 | 79513126 | SNP | T | A | T | 0.369 | 0.462 |
| rs7405590 | 79515660 | SNP | A | G | A | 0.370 | 0.460 |
| rs72854495 | 79511566 | SNP | G | A | A | 0.117 | 0.459 |
| rs7405588 | 79515636 | SNP | G | T | T | 0.117 | 0.459 |
| rs74530133 | 79516050 | SNP | C | T | C | 0.371 | 0.458 |
| rs7405749 | 79509625 | SNP | T | A | A | 0.120 | 0.450 |
| rs7406506 | 79510899 | SNP | A | G | A | 0.381 | 0.446 |
| rs7405641 | 79506890 | SNP | T | C | T | 0.382 | 0.444 |
| rs11552304 | 79514129 | SNP | G | A | G | 0.379 | 0.442 |
| rs7405532 | 79506811 | SNP | A | G | A | 0.383 | 0.442 |
| rs71675424 | 79507107 | Indel | CG | C | CG | 0.383 | 0.442 |
| rs62076028 | 79508263 | SNP | A | G | A | 0.383 | 0.442 |
| rs58483803 | 79508484 | SNP | G | A | G | 0.383 | 0.442 |
| rs11869448 | 79511379 | SNP | G | A | G | 0.380 | 0.440 |
| rs11870015 | 79511815 | SNP | C | A | C | 0.380 | 0.440 |
| rs8074089 | 79512567 | SNP | C | T | C | 0.380 | 0.440 |
| rs7405522 | 79506761 | SNP | C | G | C | 0.384 | 0.440 |
| rs7224579 | 79514832 | SNP | G | A | G | 0.380 | 0.440 |
| rs6565593 | 79515075 | SNP | A | G | A | 0.381 | 0.438 |
| rs6565590 | 79505759 | SNP | G | A | A | 0.112 | 0.437 |
| rs6565592 | 79505883 | SNP | T | G | T | 0.385 | 0.431 |
| rs72854500 | 79522734 | SNP | G | A | A | 0.113 | 0.430 |
| rs4076968 | 79519382 | SNP | G | A | G | 0.393 | 0.408 |
| rs7213717 | 79521181 | SNP | C | T | C | 0.399 | 0.398 |
| rs78537846 | 79521978 | SNP | G | A | A | 0.378 | 0.391 |
| rs9675106 | 79522024 | SNP | T | A | A | 0.380 | 0.387 |
| rs6565595 | 79521649 | SNP | G | C | G | 0.389 | 0.386 |
| rs7207933 | 79521239 | SNP | T | G | T | 0.391 | 0.382 |
| rs60016321 | 79522147 | SNP | C | T | C | 0.392 | 0.380 |
| rs112791119 | 79522032 | SNP | T | A | A | 0.378 | 0.376 |
| rs77387916 | 79510265 | SNP | G | A | A | 0.105 | 0.374 |
| rs74818865 | 79514028 | SNP | C | G | G | 0.105 | 0.374 |
| rs7207958 | 79521295 | SNP | G | A | A | 0.393 | 0.371 |
| rs12453887 | 79523741 | SNP | G | A | A | 0.391 | 0.339 |
| rs11150795 | 79524971 | SNP | G | A | A | 0.119 | 0.331 |
| rs12675 | 79507272 | SNP | G | A | A | 0.114 | 0.322 |
| rs61430049 | 79508942 | SNP | A | G | G | 0.115 | 0.317 |
| rs112930265 | 79522049 | Indel | CT | C | C | 0.434 | 0.313 |
| rs6565591 | 79505878 | SNP | C | T | C | 0.485 | 0.293 |
| rs8068081 | 79524882 | SNP | C | T | T | 0.095 | 0.291 |
| rs6565596 | 79525118 | SNP | T | G | T | 0.416 | 0.286 |
| rs8068511 | 79524790 | SNP | T | A | T | 0.418 | 0.282 |
| rs3924327 | 79518369 | SNP | A | G | A | 0.483 | 0.275 |
| rs61655749 | 79505445 | SNP | G | A | G | 0.234 | 0.233 |
| rs34797307 | 79496289 | SNP | G | A | A | 0.067 | 0.228 |
| rs35789723 | 79612621 | SNP | G | A | A | 0.078 | 0.221 |
| rs2075722 | 79503472 | SNP | A | G | G | 0.070 | 0.219 |
| rs7406756 | 79575398 | SNP | G | A | A | 0.080 | 0.219 |
| rs7406904 | 79619459 | SNP | A | T | T | 0.077 | 0.207 |

| | | | | | | | |
|---|---|---|---|---|---|---|---|
| ^{a}: based on GRCh37 genome assembly SNP: single nucleotide polymorphism, Indel (insertion/deletion): insertion/deletion, r²: linkage disequilibrium coefficient | | | | | | | |

As the gene polymorphism satisfying r² ≥ 0.8 with respect to rs11932853, 3 gene polymorphisms shown in Table 7 were identified.

**[Table 7]**

| SNP | Location^{a} | Type | Reference allele^{a} | Alternative allele ^{a} | Minor allele | Minor allele frequency | *r²* |
|---|---|---|---|---|---|---|---|
| rs13128178 | 112021897 | SNP | G | A | G | 0.454 | 1.000 |
| rs13103305 | 112021953 | SNP | T | C | T | 0.454 | 1.000 |
| rs34290584 | 112022252 | Indel | TGAGA | T | TGAGA | 0.453 | 0.996 |

| | | | | | | | |
|---|---|---|---|---|---|---|---|
| ^{a}: based on GRCh37 genome assembly SNP: single nucleotide polymorphism, Indel (insertion/deletion): insertion/deletion, r²: linkage disequilibrium coefficient | | | | | | | |

As the gene polymorphism satisfying r² ≥ 0.6 with respect to rs8032978, 28 gene polymorphisms shown in Table 8 were identified.

**[Table 8]**

| SNP | Location ^{a} | Type | Reference allele ^{a} | Alternative allele ^{a} | Minorallele | Minor allele frequency | *r²* |
|---|---|---|---|---|---|---|---|
| rs8033540 | 101800094 | SNP | A | G | G | 0.114 | 1.000 |
| rs8033003 | 101799843 | SNP | A | T | T | 0.113 | 0.990 |
| rs28863221 | 101799970 | SNP | C | T | T | 0.115 | 0.990 |
| rs28770217 | 101800037 | SNP | G | A | A | 0.115 | 0.990 |
| rs111829181 | 101798567 | Indel | CTG | C | C | 0.112 | 0.980 |
| rs55957523 | 101796090 | SNP | T | C | C | 0.111 | 0.971 |
| rs28609156 | 101795826 | SNP | C | A | A | 0.112 | 0.961 |
| rs28690028 | 101796748 | SNP | G | A | A | 0.110 | 0.961 |
| rs28665122 | 101817727 | SNP | C | T | T | 0.103 | 0.893 |
| rs7172856 | 101795153 | SNP | G | A | A | 0.096 | 0.827 |
| rs143956992 | 101807330 | SNP | T | C | C | 0.087 | 0.723 |
| rs117531330 | 101808343 | SNP | T | C | C | 0.087 | 0.723 |
| rs77343149 | 101808400 | SNP | G | C | C | 0.087 | 0.723 |
| rs74563564 | 101810403 | SNP | G | A | A | 0.088 | 0.713 |
| rs149545605 | 101812368 | SNP | T | A | A | 0.088 | 0.713 |
| rs11327127 | 101818159 | Indel | GA | G | G | 0.103 | 0.706 |
| rs117512970 | 101814405 | SNP | C | T | T | 0.087 | 0.704 |
| rs74041962 | 101823384 | SNP | C | T | T | 0.089 | 0.612 |
| rs59542966 | 101824568 | SNP | C | T | T | 0.089 | 0.612 |
| rs2898864 | 101829502 | SNP | C | G | G | 0.089 | 0.612 |
| rs4275835 | 101829666 | SNP | G | A | A | 0.089 | 0.612 |
| rs111447514 | 101829888 | SNP | G | A | A | 0.089 | 0.612 |
| rs61276520 | 101833106 | SNP | A | G | G | 0.089 | 0.612 |
| rs60105028 | 101833806 | SNP | T | C | C | 0.089 | 0.612 |
| rs75348190 | 101792183 | SNP | G | A | A | 0.078 | 0.603 |
| rs1545855 | 101831678 | SNP | C | T | T | 0.090 | 0.603 |
| rs74041979 | 101834118 | SNP | T | G | G | 0.090 | 0.603 |
| rs59199124 | 101834463 | SNP | G | A | A | 0.088 | 0.603 |

| | | | | | | | |
|---|---|---|---|---|---|---|---|
| ^{a}: based on GRCh37 genome assembly SNP: single nucleotide polymorphism, Indel (insertion/deletion): insertion/deletion, r²: linkage disequilibrium coefficient | | | | | | | |

All publications, patents and patent applications cited in the present specification are incorporated herein in their entirety by reference.

## Claims

1. A method comprising steps of: analyzing a gene polymorphism specified by one selected from the group consisting of rs9316695, rs11932853, rs28415722, rs7406710 and rs8032978 or a gene polymorphism in linkage disequilibrium or genetic linkage with the gene polymorphism present in genomic DNA of a biological sample taken from a subject; determining the genotype of the gene polymorphism; and determining a side effect from administration of trastuzumab based on the determined genotype.

2. The method according to claim 1, wherein the gene polymorphism specified by rs9316695 is located on a long arm of chromosome 13 (13q14.3) and is a single nucleotide polymorphism having cytosine as wild-type and adenine as mutant.

3. The method according to claim 1, wherein, in the gene polymorphism specified by rs9316695, a mutant is a risk allele; and it is determined that a possibility of developing a side effect of trastuzumab is low in a case of wild-type homozygosity, a possibility of developing a side effect of trastuzumab is high in a case of mutant/wild-type heterozygosity, and a possibility of developing a side effect of trastuzumab is higher in a case of mutant homozygosity.

4. The method according to claim 1, wherein the gene polymorphism specified by rs11932853 is located on a long arm of chromosome 4 (4q25) and is a single nucleotide polymorphism having thymine as wild-type and cytosine as mutant.

5. The method according to claim 1, wherein, in the gene polymorphism specified by rs11932853, a wild-type is a risk allele; and it is determined that a possibility of developing a side effect of trastuzumab is low in a case of mutant homozygosity, a possibility of developing a side effect of trastuzumab is high in a case of mutant/wild-type heterozygosity, and a possibility of developing a side effect of trastuzumab is higher in a case of wild-type homozygosity.

6. The method according to claim 1, wherein the gene polymorphism specified by rs28415722 is located on a long arm of chromosome 15 (15q26.3) and is a single nucleotide polymorphism having guanine as wild-type and adenine as mutant.

7. The method according to claim 1, wherein, in the gene polymorphism specified by rs28415722, a mutant is a risk allele; and it is determined that a possibility of developing a side effect of trastuzumab is low in a case of wild-type homozygosity, a possibility of developing a side effect of trastuzumab is low in a case of mutant/wild-type heterozygosity, and a possibility of developing a side effect of trastuzumab is higher in a case of mutant homozygosity.

8. The method according to claim 1, wherein the gene polymorphism specified by rs7406710 is located on a long arm of chromosome 17 (17q25.3) and is a single nucleotide polymorphism having cytosine as wild-type and thymine as mutant.

9. The method according to claim 1, wherein, in the gene polymorphism specified by rs7406710, a wild-type is a risk allele; and it is determined that a possibility of developing a side effect of trastuzumab is low in a case of mutant homozygosity, a possibility of developing a side effect of trastuzumab is low in a case of mutant/wild-type heterozygosity, and a possibility of developing a side effect of trastuzumab is higher in a case of wild-type homozygosity.

10. The method according to claim 1, wherein the gene polymorphism specified by rs8032978 is located on a long arm of chromosome 15 (15q26.3) and is a single nucleotide polymorphism having adenine as wild-type and guanine as mutant.

11. The method according to claim 1, wherein, in the gene polymorphism specified by rs8032978, a mutant is a risk allele; and it is determined that a possibility of developing a side effect of trastuzumab is low in a case of wild-type homozygosity, a possibility of developing a side effect of trastuzumab is high in a case of mutant/wild-type heterozygosity, and a possibility of developing a side effect of trastuzumab is higher in a case of mutant homozygosity.

12. The method according to claim 1, wherein the subject is a patient with cancer which is observed to have overexpression of HER2.

13. The method according to claim 12, wherein the cancer is breast cancer or stomach cancer.

14. The method according to claim 1, wherein the side effect is at least one selected from heart failure, cardiogenic shock, pulmonary edema, pericardial effusion, cardiomyopathy, pericarditis, arrhythmia and bradycardia.

15. A probe set for determining a side effect from administration of trastuzumab, comprising an oligonucleotide that hybridizes, under stringent conditions, with a region of consecutive 5 to 50 nucleotides containing a gene polymorphism specified by one selected from the group consisting of rs9316695, rs11932853, rs28415722, rs7406710 and rs8032978, or a gene polymorphism in linkage disequilibrium or genetic linkage with the gene polymorphism.

16. The probe set for determining a side effect according to claim 15, comprising a wild-type probe corresponding to a wild-type in the gene polymorphism and a mutant probe corresponding to a mutant in the gene polymorphism.
